# EUROPEAN PATENT SPECIFICATION

(11) **EP 1 314 741 B1**
(45) Date of publication and mention of the grant of the patent: **07.03.2007**
(21) Application number: 01127061.8
(22) Date of filing: 14.11.2001
(51) Int. Cl.: C07K 16/28, C07K 16/46, C12N 5/10, A61K 31/7088, A61P 35/02, A61K 47/38

(54) **Bispecific anti-CD19 x anti-CD16 antibodies and uses thereof**
Bispezifische Antikörper gegen CD19 und CD16 und deren Verwendung
Anticorps bispécifiques dirigés contre CD19 et CD16 et leur utilisation

(43) Date of publication of application: 28.05.2003
(73) Proprietor: Affimed Therapeutics AG, 69120 Heidelberg (DE)
(72) Inventor: Le Gall, Fabrice, 68535 Edingen-Neckarhausen (DE); Kipriyanov, M. Sergey, 69126 Heidelberg (DE); Moldenhauer, Gerhard, 69120 Heidelberg (DE); Little, Melvyn, 69151 Neckargemünd (DE); Cochlovius, Björn, 69221 Dossenheim (DE); Schäfer, J. Holger, 69117 Heidelberg (DE)
(74) Representative: Schüssler, Andrea

(56) References cited:
- EP-A- 0 952 218
- WO-A-99/57150
- HARTMANN FRANK ET AL: "Treatment of refractory Hodgkin's disease with an anti-CD16/CD30 bispecific antibody." BLOOD, vol. 89, no. 6, 1997, pages 2042-2047, XP002213356 ISSN: 0006-4971
- WEINER L M ET AL: "PHASE I TRIAL OF 2B1, A BISPECIFIC MONOCLONAL ANTIBODY TARGETING C-ERBB-2 AND FCGAMMARIII" CANCER RESEARCH, AMERICAN ASSOCIATION FOR CANCER RESEARCH, BALTIMORE, MD, US, vol. 55, no. 20, 15 October 1995 (1995-10-15), pages 4586-4593, XP002046040 ISSN: 0008-5472
- LE GALL F ET AL: "Di-, tri- and tetrameric single chain Fv antibody fragments against human CD19: effect of valency on cell binding" FEBS LETTERS, ELSEVIER SCIENCE PUBLISHERS, AMSTERDAM, NL, vol. 453, no. 1-2, 18 June 1999 (1999-06-18), pages 164-168, XP004259822 ISSN: 0014-5793
- VAN SPRIEL A B ET AL: "Immunotherapeutic perspective for bispecific antibodies" IMMUNOLOGY TODAY, ELSEVIER PUBLICATIONS, CAMBRIDGE, GB, vol. 21, no. 8, 1 August 2000 (2000-08-01), pages 391-397, XP004215167 ISSN: 0167-5699
- HUDSON P J ET AL: "High avidity scFv multimers;diabodies and triabodies" JOURNAL OF IMMUNOLOGICAL METHODS, ELSEVIER SCIENCE PUBLISHERS B.V.,AMSTERDAM, NL, vol. 231, no. 1-2, 10 December 1999 (1999-12-10), pages 177-189, XP004187644 ISSN: 0022-1759
- KIPRIYANOV SERGEY M ET AL: "Synergistic antitumor effect of bispecific CD19 X CD3 and CD19 X CD16 diabodies in a preclinical model of non-Hodgkin's lymphoma." JOURNAL OF IMMUNOLOGY, vol. 169, no. 1, 1 July 2002 (2002-07-01), pages 137-144, XP001094164 July 1, 2002 ISSN: 0022-1767

## Description

The present invention relates to multivalent multimeric antibodies comprising at least two binding sites specific for the human B cell marker CD19 and human Fc_{γ} receptor III (CD16). The present invention also relates to polynucleotides encoding said antibodies as well as vectors comprising said polynucleotides, host cells transformed therewith and their use in the production of said antibodies. Finally, the present invention relates to compositions, preferably pharmaceutical and diagnostic compositions, comprising any of above mentioned polynucleotides, antibodies or vectors. The pharmaceutical compositions are useful for immunotherapy, preferably against B cell malignancies such as non-Hodgkin's lymphoma.

Non-Hodgkin's lymphoma (NHL) encompasses a heterogeneous group of hematological malignancies of B and T cell origin occurring in blood, lymph nodes and bone marrow, which commonly disseminate throughout the body. NHL is one of the few malignancies that have increased in frequency more than the increase in population, with approximately 53,000 new cases occurring annually in the United States. The most common forms of NHL are derived from the B cell lineage. While NHL can be treated with reasonable success at early and intermediate stages, the results of conventional chemotherapy and radiation in advanced stages remain disappointing. This holds particularly true for the prevalent low-grade lymphomas. A fairly large number of patients relapse and most remissions cannot be extended beyond the minimal residual disease. This discouraging situation has stimulated the search for alternative therapeutic strategies, such as activation of host immune mechanisms using bispecific antibodies (BsAbs) (van Spriel et al., Immunol. Today 21 (2000), 391-397). The BsAb makes a bridge between the tumor cell and the immune effector cell followed by triggering the cytotoxic responses that include perforin and granzyme release, Fas-mediated apoptosis and cytokine production. Since NHLs typically express one or more B cell markers, e.g. CD19 or CD20, these markers can be used to redirect effector cells towards malignant B cells. Although normal B cells will be also destroyed, they are repopulated from stem cells lacking the targeted antigens. To mediate redirected lysis, a BsAb must bind a target cell directly to a triggering molecule on the effector. The best-studied cytotoxic triggering receptors are multichain signaling complexes, such as T cell receptor (TCR)/CD3 complex on the T cell, FcγRIIIa (CD16) on natural killer (NK) cell, FcγRI (CD64) and FcγRI (CD89) expressed by monocytes, macrophages, and granulocytes. BsAbs directed to TCR/CD3 complex have the potential to target all T cells, regardless of their natural MHC specificity. Thus far, different forms of CD19 x CD3 BsAb have been generated and used in a number of *in vitro* and *in vivo* therapeutic studies. These BsAbs have mainly been produced using rodent hybrid hybridomas or by chemical cross-linking of two monoclonal antibodies. However, the human anti-murine antibody (HAMA) response and release of inflammatory cytokines are the major drawbacks of BsAb derived from rodent antibodies in clinical use. Moreover, CD3-based immunotherapy requires additional stimulation of T cell population via a signal delivered by a distinct co-receptor. Therefore, the thus far available BsAbs suffer from low T cell cytotoxicity and the need of costimulatory agents in order to display satisfactory biological activity.

Thus, the technical problem underlying the present invention was to provide means suitable for therapy of B-cell malignancies that overcome the disadvantages of the means of the prior art.

The solution to said technical problem is achieved by providing the embodiments characterized in the claims. The present invention is based on the observation that the generation of antibodies relying on retargeting of NK cells has a positive therapeutic effect, since, unlike T cells, FcR-bearing cellular mediators of innate immunity as e.g. NK cells (and monocytes, macrophages and granulocytes) tend to exist in constitutively activated states and do not need additional (pre-)stimulation.

Furthermore, monoclonal BsAbs used so far for therapy have immunoglobulin constant domains, which are responsible for undesired immune reactions (HAMA response). Thus, a preferred embodiment of the antibodies of the present invention are BsAb which only comprise the variable immunoglobulin domains, so called Fᵥ modules by means of which undesired immune responses can be avoided. Furthermore, they have a stability that makes it usable for therapeutic uses. The Fᵥ module is formed by association of the immunoglobulin heavy and light chain variable domains, V_{H} and V_{L}, respectively. The bispecific molecule, so called bispecific diabody (BsDb), can be formed by the noncovalent association of two single-chain fusion products, consisting of the V_{H} domain from one antibody connected by a short linker to the V_{L} domain of another antibody. Alternatively, recombinant BsAb, tandem diabody (Tandab) can be formed by homodimerization of single-chain molecules comprising four antibody variable domains (V_{H} and V_{L}) of two different specificities, in an orientation preventing intramolecular Fv formation.

### Brief description of the drawings

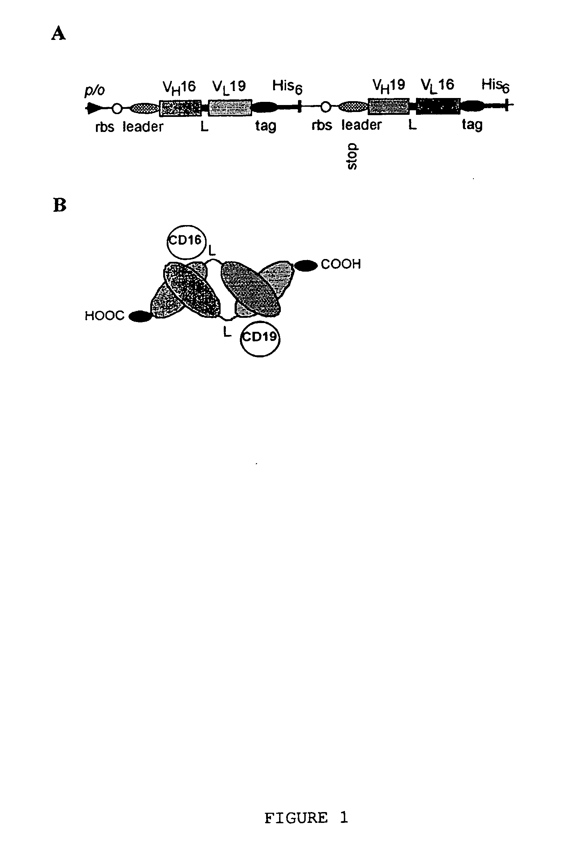

FIGURE 1: Genetic organization of operon encoding CD19 x CD16 bivalent BsDb (A) and protein model of BsDb (B)
   His₆: six C-terminal histidine residues; L: short 10 amino acid peptide linker SerAlaLysThrThrProLysLeuGlyGly connecting V_{H} and V_{L} domains; leader, bacterial leader sequence (e.g. *PelB* leader) for secretion of recombinant product into periplasm; *p*/*o*: promoter/operator; rbs, ribosome binding site; Stop: stop codon (TAA); tag: C-terminal epitope for immunodetection; V_{H} and V_{L}: variable regions of the heavy and light chains specific either to CD16 or to CD19.

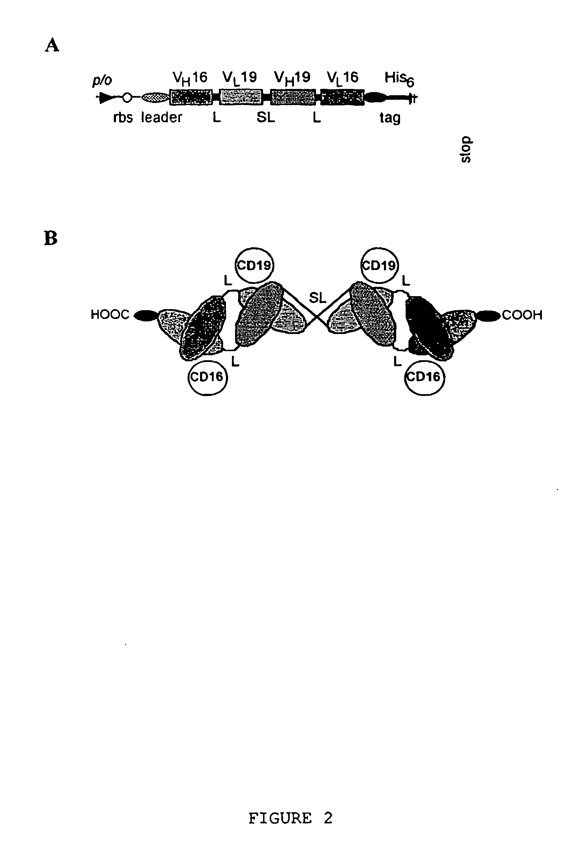

FIGURE 2: Genetic organization of operon encoding CD19 x CD16 tetravalent Tandab (A) and protein model of Tandab (B)
   His₆: six C-terminal histidine residues; L: short 10 amino acid peptide linker SerAlaLysThrThrProLysLeuGlyGly connecting V_{H} and V_{L} domains; leader, bacterial leader sequence (e.g. *Pel*B leader) for secretion of recombinant product into periplasm; *p*/*o*: promoter/operator; rbs, ribosome binding site; SL: 12 amino acid peptide linker ArgAlaAspAlaAlaAlaAlaGlyGlyProGlySer between domains in the middle of the molecule; Stop: stop codon (TAA); tag: C-terminal epitope for immunodetection; V_{H} and V_{L}: variable regions of the heavy and light chains specific either to CD16 or to CD19.

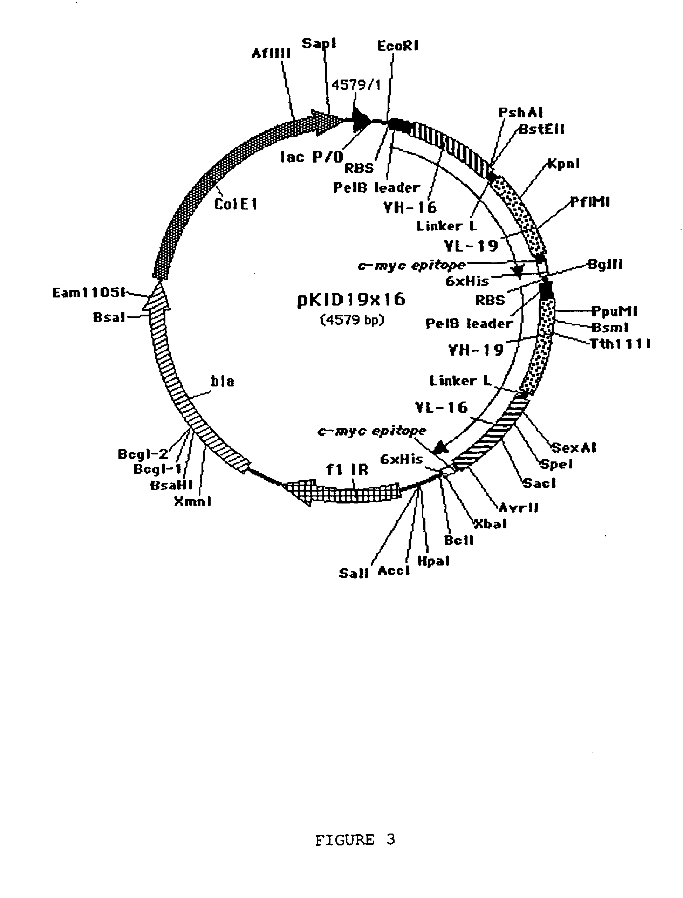

FIGURE 3: Diagram of the expression plasmid pKID19x16
   6xHis: sequence encoding six C-terminal histidine residues; bla: gene of beta-lactamase responsible for ampicillin resistance; bp: base pairs; c*-myc* epitope: sequence coding for an epitope which is recognized by the 9E10 antibody; ColE1: origin of the DNA replication; f1 IR: intergenic region of bacteriophage f1; lac P/O: wild-type *lac*-operon promoter/operator; Linker L: sequence which encodes the 10 amino acid peptide SerAlaLysThrThrProLysLeuGlyGly connecting the V_{H} and V_{L} domains; PelB leader: signal peptide sequence of the bacterial pectate lyase; RBS: *lac*Z ribosome binding site; V_{H} and V_{L}: sequence coding for the variable region of the immunoglobulin heavy and light chain, respectively. Unique restriction sites are indicated.

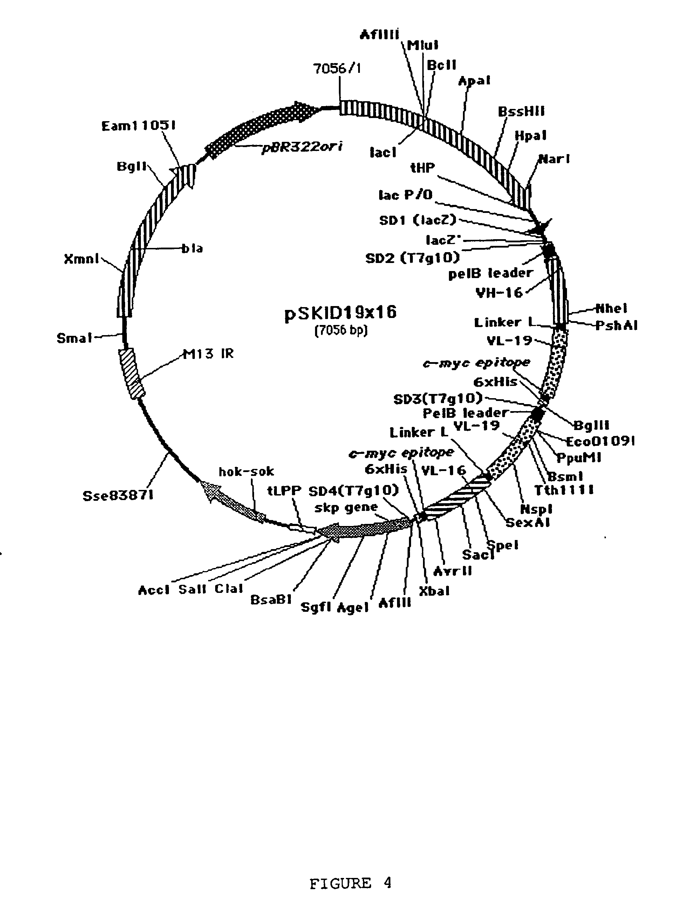

FIGURE 4: Diagram of the expression plasmid pSKID19x16
   6xHis: sequence encoding six C-terminal histidine residues; bla: gene of beta-lactamase responsible for ampicillin resistance; bp: base pairs; c-*myc* epitope: sequence coding for an epitope which is recognized by the 9E10 antibody; hok-sok: plasmid stabilizing DNA locus; lacI: gene encoding *lac-*repressor; lac P/O: wild-type lac-operon promoter/operator; Linker L: sequence which encodes the 10 amino acid peptide SerAlaLysThrThrProLysLeuGlyGly connecting the V_{H} and V_{L} domains; M13 IR: intergenic region of bacteriophage M13; pBR322ori: origin of the DNA replication; PelB leader: signal peptide sequence of the bacterial pectate lyase; SD1: Shine-Dalgarno sequence (ribosome binding site) derived from *E. coli* lacZ gene (lacZ); SD2, SD2 and SD3: Shine-Dalgarno sequence for the strongly expressed gene 10 of bacteriophage T7 (T7g10); skp gene: gene encoding bacterial periplasmic factor Skp/OmpH; tHP: strong transcriptional terminator; tLPP: lipoprotein terminator of transcription; V_{H} and V_{L}: sequence coding for the variable region of the immunoglobulin heavy and light chain, respectively. Unique restriction sites are indicated.

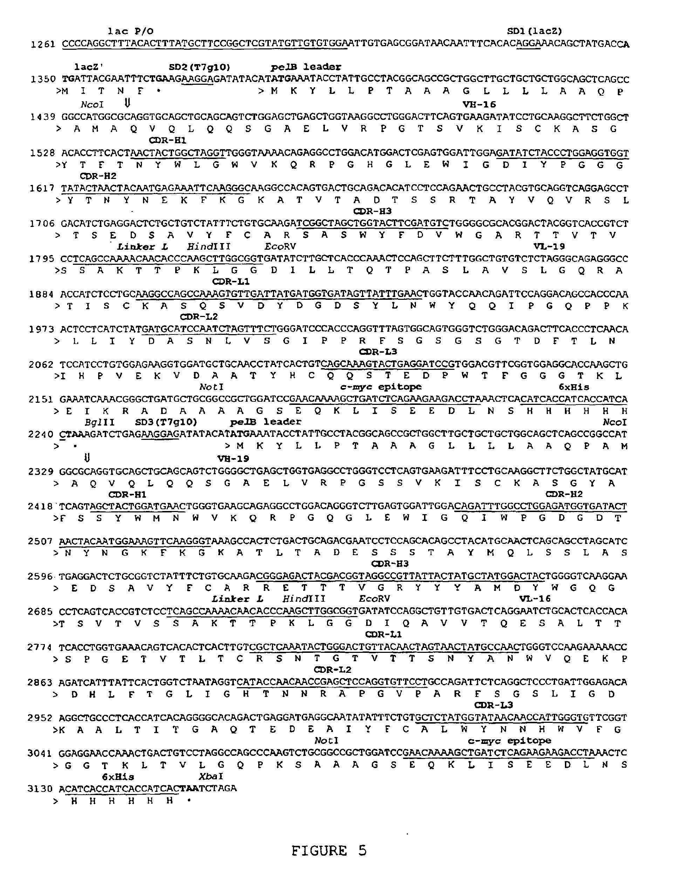

FIGURE 5: Nucleotide and deduced amino acid sequences of the CD19 x CD16 BsDb in the expression plasmid pSKID19x16
   6xHis: sequence encoding six C-terminal histidine residues; CDR: complementarity determining region of the immunoglobulin heavy (H) or light (L) chain; c-*myc* epitope: sequence coding for an epitope which is recognized by the 9E10 antibody; lac P/O: wild-type *lac*-operon promoter/operator; lacZí: gene encoding the amino terminal peptide of beta-galactosidase; Linker L: sequence which encodes the 10 amino acid peptide SerAlaLysThrThrProLysLeuGlyGly connecting the V_{H} and V_{L} domains; *pel*B leader: signal peptide sequence of the bacterial pectate lyase; SD1: Shine-Dalgarno sequence (ribosome binding site) derived from *E*. *coli* lacZ gene (lacZ); SD2, SD2 and SD3: Shine-Dalgarno sequence for the strongly expressed gene 10 of bacteriophage T7 (T7g10); V_{H} and V_{L}: sequence coding for the variable region of the immunoglobulin heavy and light chain, respectively. The restriction sites used for gene assembly are indicated. The start and stop codons of translation are shown in bold.
FIGURE 6: Analysis of purified CD19 x CD16 BsDb by size exclusion chromatography on a calibrated Superdex 200 column
   The elution positions of molecular mass standards are indicated.
FIGURE 7: Analysis of purified CD19 x CD16 BsDb by 12% sodium dodecyl sulfate-polyacrylamide gel electrophoresis (SDS-PAGE) under reducing conditions
   *Lane 1, M*ᵣ markers (kDa, *M*ᵣ in thousands); *Lane 2,* CD19 x CD16 BsDb. The gel was stained with Coomassie. Positions of hybrid V_{H}16-V_{L}19 and V_{H}19-V_{L}16 scFvs are indicated.
FIGURE 8: Lineweaver-Burk analysis of fluorescence dependence on CD19 x CD16 diabody concentration as determined by flow cytometry
   Diabody binding to CD19⁺ JOK-1 cells and CD16⁺ 293-CD16 cells was measured.
FIGURE 9: Retention of CD19 x CD16 BsDb on the surface of CD19⁺ JOK-1 cells and CD16⁺ 293-CD16 cells at 37°C
   Values are expressed as a percentage of initial mean fluorescence intensity.
FIGURE 10: Lysis of CD19⁺ Raji cells by human PBLs at different effector:target (E:T) ratios in presence of CD19 x CD16 BsDb at concentration of 0.5, 1 and 5 mg/ml
FIGURE 11: Lysis of CD19⁺ Raji cells by human NK cells at different E:T ratios in presence of CD19 x CD16 BsDb at concentration of 0.5, 1 and 5 mg/ml.
FIGURE 12: Treatment of severe combined immunodeficiency (SCID) mice bearing human Burkitt's lymphoma xenografts
   The mice received PBS (open squares), human PBLs alone (filled squares), or human PBLs followed 4 h later by the administration of CD19 x CD16 BsDb (filled circles). Tumor size was measured every second day. Tumor growth curves of individual animals till 30 day of experiment are presented.
FIGURE 13: Survival of SCID mice bearing human Burkitt's lymphoma xenografts
   The mice received PBS (open squares), human PBLs alone (filled squares) or human PBLs followed 4 h later by the administration of CD19 x CD16 BsDb (filled circles).

Accordingly, the present invention relates to a multivalent multimeric antibody, characterized by the following features:
(a) it has at least two specificities;
(b) at least one antigen-binding domain is specific to human CD19; and
(c) at least one antigen-binding domain is specific to human CD16.

The antibody of the present invention is specific to both human CD19 on malignant B cells and to human CD16 on cytotoxic NK cells. Such antibody is capable of destroying CD19-positive tumor cells by recruitment of human NK cells without any need for pre- and/or co-stimulation. This is in sharp contrast with any known bispecific molecules retargeting effector cells to CD19-positive target cells, such as CD19 x CD3 or CD19 x CD28 BsAbs. The independence from pre- and/or co-stimulation of effector cell population may substantially contribute to the therapeutic effect of such antibodies by augmentation of adverse side effects, such as cytokine release syndrome.

The antibodies of the present invention can be prepared by methods known to the person skilled in the art, e.g. by the following methods:
(a) Chemical coupling of antibodies or antibody fragments specific to human CD19 and CD16, respectively, via heterobifunctional linkers.
(b) Fusion of hybridoma cell lines which are already available and which secrete monoclonal antibodies specific to human CD19 and CD16, respectively.
(c) Transfection of the immunoglobulin light and heavy chain genes of at least two different specificities into murine myeloma cells or other eukaryotic expression systems and isolation of the polyvalent multispecific antibodies.

It is also the object of the present invention to provide an antibody by means of which undesired immune responses, such as a HAMA response, can be avoided.

Thus, in a preferred embodiment the multivalent multimeric antibody of the present invention is devoid of constant regions. This is, e.g., achieved by constructing a recombinant molecule, which consists only of non-immunogenic immunoglobulin variable (V_{H} and V_{L}) domains. This can also be achieved by using the antibody domains of human origin. Such antibodies can be prepared by methods known to the person skilled in the art, e.g. by the following methods:
(a) Construction of the multivalent multispecific single chain Fv-antibodies by combining the genes encoding at least four immunoglobulin variable V_{H} and V_{L} domains, either separated by peptide linkers or by no linkers, into a single genetic construct and expressing it in bacteria or other appropriate expression system.
(b) Non-covalent heterodimerization of two hybrid scFv fragments, each consisting of V_{H} and V_{L} domains of different specificity (against CD19 or CD16), either separated by peptide linkers or by no linkers, as a result of co-expression of corresponding genes or co-refolding of separately expressed corresponding precursors.
(c) Non-covalent homodimerization of single chain Fv-antibodies comprising at least four V_{H} and V_{L} domains of different specificity (against CD19 or CD16) either separated by peptide linkers or by no linkers, in an orientation preventing their intramolecular pairing.

In a preferred embodiment, the multivalent multimeric antibody of the present invention is a single chain Fv-antibody comprising at least four immunoglobulin variable V_{H} and V_{L} domains, either separated by peptide linkers or by no linkers.

The term "Fv-antibody" as used herein relates to an antibody containing variable domains but not constant domains. The term "peptide linker" as used herein relates to any peptide capable of connecting two variable domains with its length depending on the kinds of variable domains to be connected. The peptide linker might contain any amino acid residue with the amino acid residues glycine, serine and proline being preferred.

In a more preferred embodiment, the multivalent multimeric antibody of the present invention is a single chain Fv-antibody comprising at least four immunoglobulin variable V_{H} and V_{L} domains, either separated by peptide linkers or by no linkers. Such an antibody can be generated, e.g., by combining the genes encoding at least four immunoglobulin variable V_{H} and V_{L} domains, either separated by peptide linkers or by no linkers, into a single genetic construct and expressing it in bacteria or other appropriate expression system.

In a further more preferred embodiment, the multivalent multimeric antibody of the present invention is a heterodimer of two hybrid single chain Fv-antibodies, each consisting of V_{H} and V_{L} domains of different specificity against CD19 or CD16, either separated by peptide linkers or by no linkers. Such an antibody can be generated, e.g., by non-covalent heterodimerization of two hybrid single chain Fv-antibodies, each consisting of V_{H} and V_{L} domains of different specificity (against CD19 or CD16), either separated by peptide linkers or by no linkers, as a result of co-expression of corresponding genes or co-refolding of separately expressed corresponding precursors.

In an alternative embodiment, the multivalent multimeric antibody of the present invention is a homodimer of single chain Fv-antibodies comprising at least four V_{H} and V_{L} domains of different specificity against CD19 or CD16, either separated by peptide linkers or by no linkers.

The present invention also relates to a multivalent multimeric antibody, wherein said antigen-binding domains mimic or correspond to V_{H} and V_{L} regions from a natural antibody. Preferably, said natural antibody is a monoclonal antibody, synthetic antibody, or humanized antibody.

In a further preferred embodiment, the multivalent multimeric antibody of the present invention is an antibody, wherein (a) the first hybrid single chain Fv-antibody is V_{H}16-V_{L}19 and the second hybrid single chain Fv-antibody is V_{H}19-V_{L}16; or (b) the first hybrid single chain Fv-antibody is V_{L}16-V_{H}19 and the second hybrid single chain Fv-antibody is V_{L}19-V_{H}16.

Preferably, the peptide linker connecting the variable V_{H} and V_{L} domains comprises comprises 0 to 12 amino acids, preferably 10 amino acids.

In a further preferred embodiment, the variable V_{H} or V_{L} domains of the multivalent multimeric antibody are shortened by at least one amino acid residue at their N- and/or C-terminus.

In a more preferred embodiment, the multivalent multimeric antibody of the present invention is an antibody wherein (a) the first hybrid single chain Fv-antibody is V_{H}16-V_{L}19 and the second hybrid single chain Fv-antibody is V_{H}19-V_{L}16; or (b) the first hybrid single chain Fv-antibody is V_{L}16-V_{H}19 and the second hybrid single chain Fv-antibody is V_{L}19-V_{H}16 and wherein the two hybrid single chain Fv-antibodies are connected via a peptide linker. A preferred length of this peptide linker is 0-30 amino acids with a length of 12 amino acid being more preferred.

The peptide linkers of the multivalent multimeric antibodies of the present invention might comprise any amino acid residue, however alanine, glycine, serine and proline residues are preferred.

The non-covalent binding of the multivalent multimeric antibody of the present invention can be strengthened by the introduction of at least one disulfide bridge between at least one pair of V-domains. This can be achieved by modifying the DNA sequences encoding the variable domains accordingly, i.e. by inserting in each of the DNA sequences encoding the two domains a codon encoding a cysteine residue or by replacing a codon by a codon encoding a cysteine residue.

Finally, the multivalent multimeric antibody of the present invention can be further modified using conventional techniques known in the art, for example, by using amino acid deletion(s), insertion(s), substitution(s), addition(s), and/or recombination(s) and/or any other modification(s) known in the art either alone or in combination. Methods for introducing such modifications in the DNA sequence underlying the amino acid sequence of a variable domain or peptide linker are well known to the person skilled in the art; see, e.g., Sambrook, Molecular Cloning A Laboratory Manual, Cold Spring Harbor Laboratory (1989) N.Y.

The multivalent multimeric antibodies of the present invention can comprise at least one further compound, e.g., a protein domain, said protein domain being linked by covalent or non-covalent bonds. The linkage can be based on genetic fusion according to the methods known in the art and described above or can be performed by, e.g., chemical cross-linking as described in, e.g., WO 94/04686. The additional domain present in the fusion protein comprising the antibody employed in accordance with the invention may preferably be linked by a flexible linker, advantageously a peptide linker, wherein said peptide linker comprises plural, hydrophilic, peptide-bonded amino acids of a length sufficient to span the distance between the C-terminal end of said further protein domain and the N-terminal end of the antibody or vice versa. The above described fusion protein may further comprise a cleavable linker or cleavage site for proteinases. Thus, e.g., at least one monomer of the antibody might be linked to a an effector molecule having a conformation suitable for biological actvity or selective binding to a solid support, a biologically active substance (e.g. a cytokine or growth hormone), a chemical agent (e.g. doxorubicin, cyclosporin), a peptide (e.g. α-amanitin), a protein (e.g. granzyme A and B) or a drug.

Another object of the present invention is a process for the preparation of a multivalent multimeric Fv-antibody according to the present invention, wherein (a) DNA sequences encoding the peptid linkers are ligated with the DNA sequences encoding the variable domains such that the peptide linkers connect the variable domains resulting in the formation of a DNA sequence encoding a monomer of the multivalent multimeric Fv-antibody and (b) the DNA sequences encoding the various monomers are expressed in a suitable expression system. The various steps of this process can be carried according to standard methods, e.g. methods described in Sambrook et al., or described in the Examples, below.

The present invention also relates to a polynucleotide encoding the multivalent multimeric antibody of the present invention and vectors, preferably expression vectors containing said polynucleotides.

A variety of expression vector/host systems may be utilized to contain and express sequences encoding the multivalent multimeric antibody. These include, but are not limited to, microorganisms such as bacteria transformed with recombinant bacteriophage, plasmid, or cosmid DNA expression vectors; yeast transformed with yeast expression vectors; insect cell systems infected with virus expression vectors (e.g., baculovirus); plant cell systems transformed with virus expression vectors (e.g., cauliflower mosaic virus, CaMV; tobacco mosaic virus, TMV) or with bacterial expression vectors (e.g., Ti or pBR322 plasmids); or animal cell systems. The invention is not limited by the host cell employed.

The "control elements" or "regulatory sequences" are those non-translated regions of the vector-enhancers, promoters, 5' and 3' untranslated regions which interact with host cellular proteins to carry out transcription and translation. Such elements may vary in their strength and specificity. Depending on the vector system and host utilized, any number of suitable transcription and translation elements, including constitutive and inducible promoters, may be used. For example, when cloning in bacterial systems, inducible promoters such as the hybrid lacZ promoter of the Bluescript.RTM. phagemid (Stratagene, LaJolla, Calif.) or pSport1.TM. plasmid (Gibco BRL) and the like may be used. The baculovirus polyhedrin promoter may be used in insect cells. Promoters or enhancers derived from the genomes of plant cells (e.g., heat shock, RUBISCO; and storage protein genes) or from plant viruses (e.g., viral promoters or leader sequences) may be cloned into the vector. In mammalian cell systems, promoters from mammalian genes or from mammalian viruses are preferable. If it is necessary to generate a cell line that contains multiple copies of the sequence encoding the multivalent multimeric antibody, vectors based on SV40 or EBV may be used with an appropriate selectable marker.

In bacterial systems, a number of expression vectors may be selected depending upon the use intended for the multivalent multimeric antibody. Vectors suitable for use in the present invention include, but are not limited to the pSKK expression vector for expression in bacteria.

In the yeast, Saccharomyces cerevisiae, a number of vectors containing constitutive or inducible promoters such as alpha factor, alcohol oxidase, and PGH may be used. For reviews, see Grant et al. (1987) Methods Enzymol. 153:516-544.

In cases where plant expression vectors are used, the expression of sequences encoding the multivalent multimeric antibody may be driven by any of a number of promoters. For example, viral promoters such as the 35S and 19S promoters of CaMV may be used alone or in combination with the omega leader sequence from TMV (Takamatsu, N. (1987) EMBO J. 6:307-311). Alternatively, plant promoters such as the small subunit of RUBISCO or heat shock promoters may be used (Coruzzi, G. et al. (1984) EMBO J. 3:1671-1680; Broglie, R. et al. (1984) Science 224:838-843; and Winter, J. et al. (1991) Results Probl. Cell Differ. 17:85-105). These constructs can be introduced into plant cells by direct DNA transformation or pathogen-mediated transfection. Such techniques are described in a number of generally available reviews (see, for example, Hobbs, S. and Murry, L. E. in McGraw Hill Yearbook of Science and Technology (1992) McGraw Hill, New York, N.Y.; pp. 191-196.

An insect system may also be used to express the multivalent multimeric antibody of the present invention. For example, in one such system, Autographa californica nuclear polyhedrosis virus (AcNPV) is used as a vector to express foreign genes in Spodoptera frugiperda cells or in Trichoplusia larvae. The sequences encoding the multivalent multimeric antibody may be cloned into a non-essential region of the virus, such as the polyhedrin gene, and placed under control of the polyhedrin promoter. Successful insertion of the gene encoding the multivalent multimeric antibody will render the polyhedrin gene inactive and produce recombinant virus lacking coat protein. The recombinant viruses may then be used to infect, for example, S. frugiperda cells or Trichoplusia larvae in which APOP may be expressed (Engelhard, E. K. et al. (1994) Proc. Nat. Acad. Sci. 91:3224-3227).

In mammalian host cells, a number of viral-based expression systems may be utilized. In cases where an adenovirus is used as an expression vector, sequences encoding the multivalent multimeric antibody may be ligated into an adenovirus transcription/translation complex consisting of the late promoter and tripartite leader sequence. Insertion in a non-essential E1 or E3 region of the viral genome may be used to obtain a viable virus which is capable of expressing the multivalent multimeric antibody in infected host cells (Logan, J. and Shenk, T. (1984) Proc. Natl. Acad. Sci. 81:3655-3659). In addition, transcription enhancers, such as the Rous sarcoma virus (RSV) enhancer, may be used to increase expression in mammalian host cells.

Human artificial chromosomes (HACs) may also be employed to deliver larger fragments of DNA than can be contained and expressed in a plasmid. HACs of 6 to 10M are constructed and delivered via conventional delivery methods (liposomes, polycationic amino polymers, or vesicles) for therapeutic purposes.

Specific initiation signals may also be used to achieve more efficient translation of sequences encoding the multivalent multimeric antibody. Such signals include the ATG initiation codon and adjacent sequences. In cases where sequences encoding the multivalent multimeric antibody, its initiation codon, and upstream sequences are inserted into the appropriate expression vector, no additional transcriptional or translational control signals may be needed. However, in case where only coding sequence is inserted, exogenous translational control signals including the ATG initiation codon should be provided. Furthermore, the initiation codon should be in the correct reading frame to ensure translation of the entire insert. Exogenous translational elements and initiation codons may be of various origins, both natural and synthetic. The efficiency of expression may be enhanced by the inclusion of enhancers which are appropriate for the particular cell system which is used, such as those described in the literature (Scharf, D. et al. (1994) Results Probl. Cell Differ. 20:125-162).

In addition, a host cell strain may be chosen for its ability to modulate the expression of the inserted sequences or to process the expressed antibody chains in the desired fashion. Post-translational processing which cleaves a "prepro" form of the protein may also be used to facilitate correct insertion, folding and/or function. Different host cells which have specific cellular machinery and characteristic mechanisms for post-translational activities (e.g., CHO, HeLa, MDCK, HEK293, and W138), are available from the American Type Culture Collection (ATCC; Bethesda, Md.) and may be chosen to ensure the correct modification and processing of the foreign antibody chains.

For long-term, high-yield production of recombinant antibodies, stable expression is preferred. For example, cell lines which stably express the multivalent multimeric antibody may be transformed using expression vectors which may contain viral origins of replication and/or endogenous expression elements and a selectable marker gene on the same or on a separate vector. Following the introduction of the vector, cells may be allowed to grow for 1-2 days in an enriched media before they are switched to selective media. The purpose of the selectable marker is to confer resistance to selection, and its presence allows growth and recovery of cells which successfully express the introduced sequences. Resistant clones of stably transformed cells may be proliferated using tissue culture techniques appropriate to the cell type.

Any number of selection systems may be used to recover transformed cell lines. These include, but are not limited to, the herpes simplex virus thymidine kinase (Wigler, M. et al. (1977) Cell 11:223-32) and adenine phosphoribosyltransferase (Lowy, I. et al. (1980) Cell 22:817-23) genes which can be employed in tk.sup.- or aprt.sup.- cells, respectively. Also, antimetabolite, antibiotic or herbicide resistance can be used as the basis for selection; for example, dhfr which confers resistance to methotrexate (Wigler, M. et al. (1980) Proc. Natl. Acad. Sci. 77:3567-70); npt, which confers resistance to the aminoglycosides neomycin and G-418 (Colbere-Garapin, F. et al (1981) J. Mol. Biol. 150:1-14) and als or pat, which confer resistance to chlorsulfuron and phosphinotricin acetyltransferase, respectively (Murry, supra). Additional selectable genes have been described, for example, trpB, which allows cells to utilize indole in place of tryptophan, or hisD, which allows cells to utilize histinol in place of histidine (Hartman, S. C. and R. C. Mulligan (1988) Proc. Natl. Acad. Sci. 85:8047-51). Recently, the use of visible markers has gained popularity with such markers as anthocyanins, beta-glucuronidase and its substrate GUS, and luciferase and its substrate luciferin, being widely used not only to identify transformants, but also to quantify the amount of transient or stable protein expression attributable to a specific vector system (Rhodes, C. A. et al. (1995) Methods Mol. Biol. 55:121-131).

A particular preferred expression vector is pSKID19x16 deposited with the DSMZ (Deutsche Sammlung für Mikroorganismen und Zellen) according to the Budapest Treaty under DSM 14529 on September 24, 2001.

The present invention also relates to a composition containing a multivalent multimeric antibody of the present invention, a polynucleotide or an expression vector. Preferably, said composition is a pharmaceutical composition preferably combined with a suitable pharmaceutical carrier or a diagnostic composition optionally further comprising suitable means for detection. Examples of suitable pharmaceutical carriers are well known in the art and include phosphate buffered saline solutions, water, emulsions, such as oil/water emulsions, various types of wetting agents, sterile solutions etc.. Such carriers can be formulated by conventional methods and can be administered to the subject at a suitable dose. Administration of the suitable compositions may be effected by different ways, e.g. by intravenous, intraperetoneal, subcutaneous, intramuscular, topical or intradermal administration. The route of administration, of course, depends on the nature of the disease, e.g. tumor, and the kind of compound contained in the pharmaceutical composition. The dosage regimen will be determined by the attending physician and other clinical factors. As is well known in the medical arts, dosages for any one patient depends on many factors, including the patient's size, body surface area, age, sex, the particular compound to be administered, time and route of administration, the kind of the disorder, general health and other drugs being administered concurrently.

Preferred medical uses of the compounds of the present invention described above are the treatment of a B-cell malignancy, preferably non-Hodgkin disease, a B-cell mediated autoimmune disease or a depletion of B-cells.

Another subject matter of the present invention relates to a diagnostic kit, comprising: (a) a multivalent multimeric antibody according to the invention, (b) an expression vector according to the invention, and, optionally, (c) conventional auxiliary agents, such as buffers, solvents and controls.

The multivalent multimeric antibody can be detectably labeled. In a preferred embodiment, said kit allows diagnosis by ELISA and contains the antibody bound to a solid support, for example, a polystyrene microtiter dish or nitrocellulose paper, using techniques known in the art. Alternatively, said kit is based on a RIA and contains said antibody marked with a radioactive isotope. In a preferred embodiment of the kit of the invention the multivalent multimeric antibody is labelled with enzymes, fluorescent compounds, luminescent compounds, ferromagnetic probes or radioactive compounds.

The below examples explain the invention in more detail.

### Example 1: Construction of the plasmids pKID19x16 and pSKID19x16 for the expression of bivalent bispecific CD19 x CD16 diabody in bacteria

The genes coding for V_{H}16-V_{L}19 and V_{H}19-V_{L}16 hybrid scFvs were constructed by exchange of the anti-CD3 V_{H} and V_{L} genes in plasmids pHOG3-19 and pHOG19-3 (Kipriyanov et al., 1998, *Int. J. Cancer* 77, 763-772) for their anti-human CD16 counterparts (Arndt et al., 1999, *Blood* 94 2562-2568) using *Nco*I/*Hind*III and *Hind*III/*Xba*I restriction sites, respectively. The expression plasmid pKID19x16 containing dicistronic operon for cosecretion of two hybrid scFv was constructed by ligation of the *Bgl*II/*Xba*I restriction fragment from pHOG16-19 comprising the vector backbone and the *Bgl*II/*Xba*I fragment from pHOG19-16 (FIGURE 3).

To increase the yield of functional CD19 x CD16 BsDb in the bacterial periplasm, an optimized expression vector pSKID19x16 was generated (FIGURE 4). This vector was constructed on the base of plasmid pHKK (Horn et al., 1996, *Appl. Microbiol.*

*Biotechnol.* 46, 524-532) containing *hok*/*sok* plasmid-free cell suicide system (Thisted et al., 1994, *EMBO J.* 13, 1960-1968). First, the gene coding for hybrid scFv V_{H}3-V_{L}19 was amplified by PCR from the plasmid pHOG3-19 (Kipriyanov et al., 1998, *Int. J. Cancer* 77, 763-772) using the primers 5-NDE, 5'-GATATACATATGAAATACCTATTGCCTACGGC-3', and 3-AFL, 5'-CGAATTCTTAAGTTAGCACAGGCCTCTAGAGACACACAGATCTTTAG-3'. The resulting 921 bp PCR fragment was digested with *Nde*I and *Afl*II and cloned into the *Nde*I/*Afl*II linearized plasmid pHKK generating the vector pHKK3-19. To delete an extra *Xba*I site, a fragment of pHKK plasmid containing 3'-terminal part of the *lac*I gene (encodes the *lac* repressor), the strong transcriptional terminator t_{HP} and wild-type *lac* promoter/operator was amplified by PCR using primers 5-NAR, 5'-CACCCTGGCGCCCAATACGCAAACCGCC-3', and 3-NDE, 5'-GGTATTTCATATGTATATCTCCTTCTTCAGAAATTCGTAATCATGG-3'. The resulting 329 bp DNA fragment was digested with NarI and NdeI and cloned into *Na*rI/*Nde*I linearized plasmid pHKK3-19 generating the vector pHKKDXba. To introduce a gene encoding the Skp/OmpH periplasmic factor for higher recombinant antibody production(Bothmann and Plückthun, 1998, *Nat. Biotechnol*. 16, 376-380), the *skp* gene was amplified by PCR with primers skp-3, 5'-CGAATTCTTAAGAAGGAGATATACATATGAAAAAGTGGTTATTAGCTGCAGG-3' and skp-4, 5'-CGAATTCTCGAGCATTATTTAACCTGTTTCAGTACGTCGG-3' using as a template the plasmid pGAH317 (Holck and Kleppe, 1988, *Gene* 67, 117-124). The resulting 528 bp PCR fragment was digested with *Afl*II and *Xho*I and cloned into the *Afl*II/*Xho*I digested plasmid pHKKDXba resulting in the expression plasmid pSKK2.

The gene encoding the hybrid scFv V_{H}16-V_{L}19 was isolated as 820 bp DNA fragment after digestion of pHOG16-19 with *Nco*I and *Xba*I*.* This gene was cloned into *Nco*I/*Xba*I linearized vector pSKK2 resulting in plasmid pSKK16-19. The gene encoding the second hybrid scFv V_{H}19-V_{L}16 was amplified from the plasmid pHOG19-16 by PCR with the primers 5-BGL, 5'-GCACACAGATCTGAGAAGGAGATATACATATGAAATACCTATTGCCTACGGC-3', and pSEXBn, 5'-GGTCGACGTTAACCGACAAACAACAGATAAAACG-3'. The resulting PCR fragment was digested with *Bgl*II and *Xba*I and cloned into the *Bgl*II/*Xba*I linearized plasmid pSKK16-19 generating the expression vector pSKID19x16 (FIGURE 4). This vector contains several features that improve plasmid performance and lead to increased accumulation of functional bivalent product in the *E. coli* periplasm under conditions of both shake-flask cultivation and high cell density fermentation. These are the *hok*/*sok* post-segregation killing system, which prevents plasmid loss, strong tandem ribosome-binding sites and a gene encoding the periplasmic factor Skp/OmpH that increases the functional yield of antibody fragments in bacteria. The expression cassette is under the transcriptional control of the wt *lac* promoter/operator system and includes a short sequence coding for the N-terminal peptide of beta-galactosidase (*lacZí*) with a first rbs derived from the *E. coli lac*Z gene, followed by genes encoding the two hybrid scFvs and Skp/OmpH periplasmic factor under the translational control of strong rbs from gene 10 of phage T7 (T7g10).

The nucleotide and protein sequences of the CD19 x CD16 BsDb in plasmid pSKID19x16 are indicated in FIGURE 5.

### Example 2: Production in bacteria and purification of bivalent bispecific CD19 x CD16 diabody

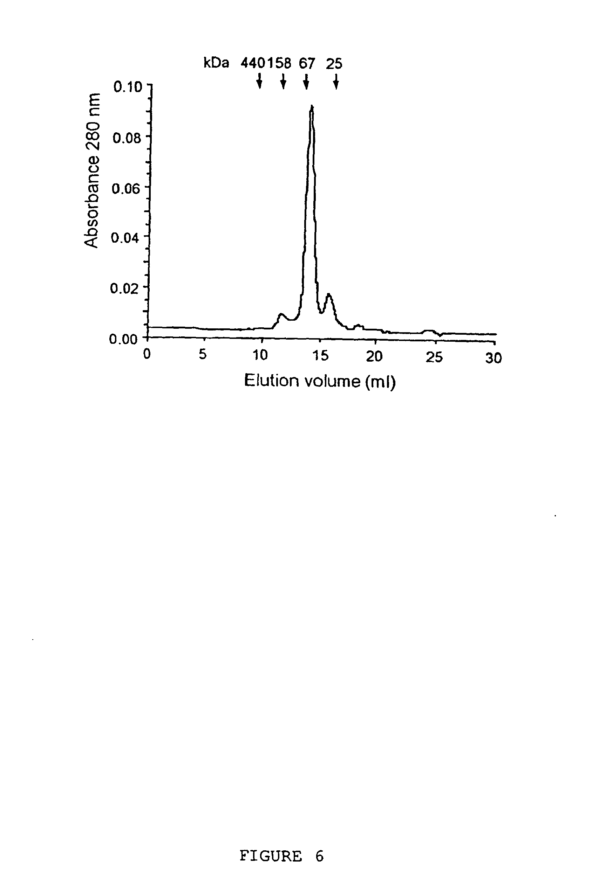

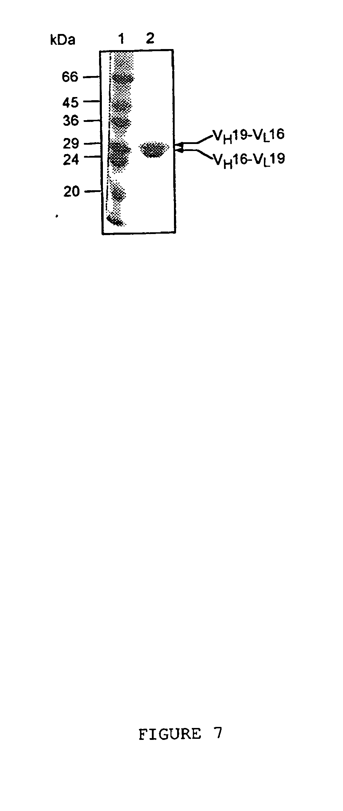

The *E. coli* XL1-Blue cells (Stratagene, La Jolla, CA) transformed with the expression plasmid pKID19x16 or *E. coli* strain RV308 (Maurer *et al*., 1980, *J. Mol. Biol.* 139, 147-161) transformed with the expression plasmid pSKID19x16 were grown overnight in 2xYT medium with 50 µg/ml ampicillin and 100 mM glucose (2xYT_{GA}) at 37°C or 26°C, respectively. Dilutions (1:50) of the overnight cultures in 2xYT_{GA} were grown as flask cultures at 37°C (XL1-Blue) or at 26°C (RV308) with vigorous shaking (180-220 rpm) until optical density (OD) 600 nm reached 0.8-0.9. Bacteria were harvested by centrifugation at 5,000 *g* for 10 min at 20°C and resuspended in the same volume of fresh YTBS medium (2xYT containing 1 M sorbitol, 2.5 mM glycine betaine and 50 µg/ml ampicillin. Isopropyl-β-D-thiogalactopyranoside (IPTG) was added to a final concentration of 0.2 mM and growth was continued at 20°C for 16-18 h. Cells were harvested by centrifugation at 9,000 g for 20 min and 4°C. In case of using RV308/pSKID19x16, the culture supernatant was discarded. In contrast, the supernatant was retained and kept on ice for XL1-Blue/pKID19x16. To isolate soluble periplasmic proteins, the pelleted bacteria were resuspended in 5% of the initial volume of ice-cold 50 mM Tris-HCl, 20% sucrose, 1 mM EDTA, pH 8.0. After 1 h incubation on ice with occasional stirring, the spheroplasts were centrifuged at 30,000 g for 30 min and 4°C leaving the soluble periplasmic extract as the supernatant and spheroplasts plus the insoluble periplasmic material as the pellet. For RV308/pSKID19x16, periplasmic extract was thoroughly dialyzed against 50 mM Tris-HCl, 1 M NaCl, pH 7.0, and used as a starting material for isolating CD19 x CD16 BsDb. For XL1-Blue/pKID19x16, the culture supernatant and the soluble periplasmic extract were combined and clarified by additional centrifugation (30,000 *g*, 4°C, 40 min). The recombinant product was concentrated by ammonium sulfate precipitation (final concentration 70% of saturation). The protein precipitate was collected by centrifugation (10,000 g, 4°C, 40 min) and dissolved in 10% of the initial volume of 50 mM Tris-HCl, 1 M NaCl, pH 7.0 (starting material). Immobilized metal affinity chromatography (IMAC) was performed at 4°C using a 5 ml column of Chelating Sepharose (Amersham Pharmacia, Freiburg, Germany) charged with Cu²⁺ and equilibrated with 50 mM Tris-HCl, 1 M NaCl, pH 7.0 (start buffer). The sample was loaded by passing the sample over the column. It was then washed with twenty column volumes of start buffer followed by start buffer containing 50 mM imidazole until the absorbance (280 nm) of the effluent was minimal (about thirty column volumes). Absorbed material was eluted with 50 mM Tris-HCl, 1 M NaCl, 250 mM imidazole, pH 7.0. The final purification was achieved by ion-exchange chromatography on a Mono Q HR 5/5 column (Amersham Pharmacia) in 20 mM Tris-HCl, pH 8.5 with a linear 0-1 M NaCl gradient. The fractions containing diabody were concentrated with simultaneous buffer exchange for PBS containing 50 mM imidazole, pH 7.0 using Ultrafree-15 centrifugal filter device (Millipore, Eschborn, Germany). Protein concentrations were determined by the Bradford dye-binding assay (Bradford, 1976, *Anal. Biochem*., 72, 248-254) using the Bio-Rad (Munich, Germany) protein assay kit. Purified CD19 x CD16 BsDb was mainly in a dimeric form with a molecular mass (*M*ᵣ) around 60 kDa, as demonstrated by gel filtration on a calibrated Superdex 200 HR 10/30 column (Amersham Pharmacia) (FIGURE 6). SDS-PAGE analysis demonstrated that the BsDb could be resolved into two protein bands corresponding to the calculated *M*ᵣ of 28,730 for V_{H}16-V_{L}19 scFv and *M*ᵣ of 29,460 for V_{H}19-V_{L}16 scFv (FIGURE 7).

### Example 3: Determination of diabody affinity by surface plasmon resonance (SPR).

Kinetic constants of interaction of CD19 x CD16 BsDb with extracellular domain (ECD) of human CD16 were determined by SPR using BIAcore 2000 biosensor system (Biacore, Uppsala, Sweden). For immobilization on a streptavidin coated sensor chip SA (Biacore), the CD16 ECD was biotinylated according to a modified protocol of the ECL protein biotinylation module (Amersham Pharmacia). As a negative control, biotinylated porcine tubulin was used. The biotinylated antigens diluted in HBS-EP buffer (10 mM HEPES, 0.15 M NaCl, 3 mM EDTA, 0.005% polyoxyethylenesorbitan; Biacore) at a concentration of 10 µg/ml were applied to a sensor chip at a flow rate of 5 µl/min for 4 min resulting in immobilization of 800 resonance units (RU) of CD16 ECD and 900 RU of tubulin. All SPR measurements were carried out at a flow rate of 20 µl/min in HBS-EP at 25°C. Analyses were performed at eight diabody concentrations from 6.25 to 800 nM. Each injected sample (100 µl) was in contact with immobilized antigen for 5 min. The dissociation was followed for 10 min. After each cycle, the surface of the sensor chip was flushed with the buffer. Kinetic constants were calculated according to 1:1 (Langmuir) binding model using BIAevaluation version 3.0 software (Biacore). CD19 x CD16 BsDb exhibited a fairly high off-rate from CD16 coated sensor chip, thus making the regeneration of the biosensor surface unnecessary. The calculated off- and on-rate constants were 2.3 x 10⁻² s⁻¹ and 2.7 x 10⁴ s⁻¹ M⁻¹, respectively, resulting in a *K*_{d} of 8.5 x 10⁻⁷ M (Table 1). A nearly identical affinity constant was deduced from the evaluation of steady-state binding levels (Table 1).

**Table 1**

| **Affinity and kinetics of BsDb CD19 x CD16 binding to CD16 ECD as determined by SPR** | | | | |
|---|---|---|---|---|
| *k*ₒₙ (M⁻¹s⁻¹) | *k*_{off} (s⁻¹) | *t*_{1/2} (min) | *K*_{d} (M) | *K*_{eq} (M) |
| 2.7 x 10⁴ | 2.3 x 10⁻² | 0.5 | 8.5 x 10⁻⁷ | 8.7 x 10⁻⁷ |

*k*_{off} and *k*ₒₙ values were measured by SPR using immobilized biotinylated CD16 ECD. Affinity constants were calculated either from the ratio *k*_{off}/*k*ₒₙ (*K*_{d}) or from the steady-state analysis of SPR data (*K*_{eq}). The half-life (*t*_{1/2}) for dissociation of diabody-antigen complex was deduced from the ratio ln2/*k*_{off}.

### Example 4: Cell binding measurements

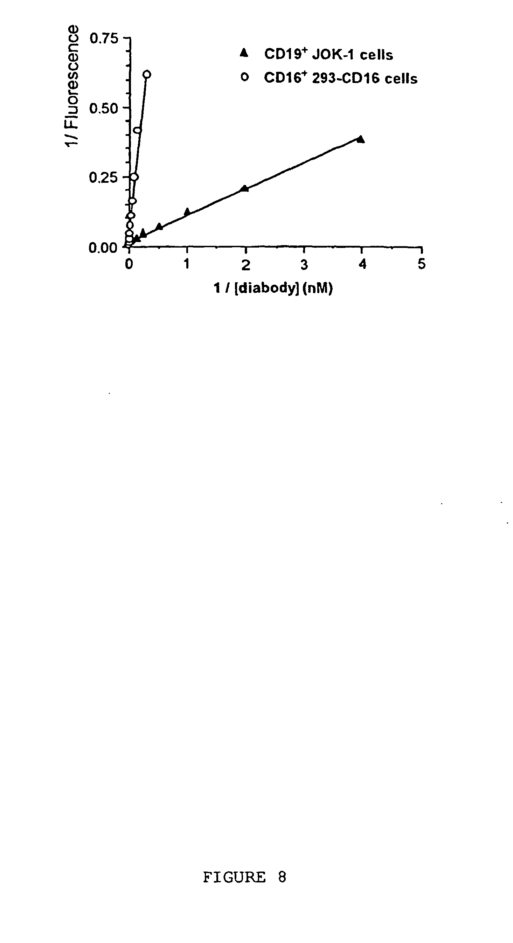

The human CD19⁺ B cell line JOK-1 and the human embryonic kidney (HEK) 293 cells stably transfected with human CD16B cDNA (293-CD16) cells were used for flow cytometry experiments. In brief, 5 x 10⁵ cells in 50 µl RPMI 1640 medium (GIBCO BRL, Eggestein, Germany) supplemented with 10% FCS and 0.1% sodium azide (referred to as complete medium) were incubated with 100 ml of diabody preparation for 45 min on ice. After washing with complete medium, the cells were incubated with 100 µl of 10 µg/ml anti-c-*myc* MAb 9E10 in the same buffer for 45 min on ice. After a second washing cycle, the cells were incubated with 100 µl of FITC-labeled goat anti-mouse IgG (GIBCO BRL) under the same conditions as before. The cells were then washed again and resuspended in 100 µl of 1 µg/ml solution of propidium iodide (Sigma, Deisenhofen, Germany) in complete medium to exclude dead cells. Fluorescence of stained cells was measured using a FACScan flow cytometer (Becton Dickinson, Mountain View, CA). Mean fluorescence (F) was calculated using Cellquest software (Becton Dickinson) and the background fluorescence was subtracted. Equilibrium dissociation constants (*K*_{eq}) were determined by fitting the experimental values to the Lineweaver-Burk equation: 1/F = 1/Fₘₐₓ + (*K*_{eq}/Fₘₐₓ) (1/[BsDb]) using the software program GraphPad Prism (GraphPad Software, San Diego, CA). The flow cytometry experiments demonstrated a specific interaction of CD19 x CD16 BsDb with both CD19⁺ JOK-1 cells and 293-CD16 cells expressing ECD of human CD16 on their surface. However, the fluorescence intensities obtained for interaction with JOK-1 cells were significantly higher than for 293-CD16 cells reflecting the 6-fold difference in affinity values for the two antigen-binding sites (FIGURE 8, Table 2).

### Example 5: In vitro cell surface retention

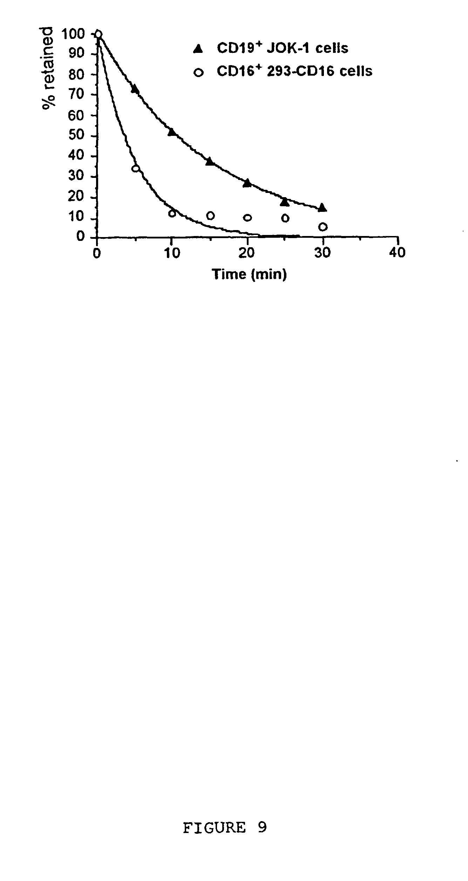

To investigate the biological relevance of the differences in direct binding experiments, the *in vitro* retention of the CD19 x CD16 BsDb on the surface of both CD19⁺ and CD16⁺ cells at 37°C was determined by flow cytometry (FIGURE 9). Cell surface retention assays were performed at 37°C under conditions preventing internalization of cell surface antigens, as described (Adams et al., 1998, *Cancer Res.* 58, 485-490),except that the detection of retained diabody was performed using anti-c-*myc* MAb 9E10 (IC Chemikalien) followed by FITC-labeled anti-mouse IgG (GIBCO BRL). Kinetic dissociation constant (*k*_{off}) and half-life (*t*_{1/2}) values for dissociation of diabody were deduced from a one-phase exponential decay fit of experimental data using GraphPad Prism (GraphPad Software). CD19 x CD16 BsDb had a relatively short retention half-life (*t*_{1/2}) on 293-CD16 cells (3.6 min) and 3-fold longer *t*_{1/2} on the surface of CD19⁺ JOK-1 cells, thus correlating well with the lower CD16 binding affinity deduced from direct binding experiments (Table 2). To determine whether the CD19 activity of diabody is influenced by the second moiety of the bispecific molecule, CD19 x CD3 BsDb (Cochlovius et al., 2000, *J. Immunol.* 165, 888-895; Kipriyanov et al., 1998, *Int. J. Cancer* 77, 763-772) was used as a control in all flow cytometry experiments. Direct binding and cell surface retention on CD19⁺ JOK-1 cells were practically indistinguishable for both diabodies. The calculated *K*_{eq} and *t*_{1/2} values were 5.7 nM and 10.8 min, respectively, for CD19 x CD3 BsDb, and 6.1 nM and 10.6 min for CD19 x CD16 BsDb. These results indicate that the second specificity present in the bispecific diabody does not significantly affect the affinity for binding to CD19.

**Table 2**

| **Affinity and kinetics of BsDb CD19 x CD16 binding to cell surface antigens** | | | |
|---|---|---|---|
| Cells | *k*_{off} (s⁻¹) | *t*_{1/2}(min) | *K*_{eq} (M) |
| JOK-1 (CD19⁺) | 1.1 x 10⁻³ | 10.6 | 6.1 x 10⁻⁹ |
| 293-CD16 (CD16⁺) | 3.2 x 10⁻³ | 3.6 | 3.9 x 10⁻⁸ |

*k*_{off} values were deduced from JOK-1 and 293-CD16 cell surface retention experiments. Equilibrium dissociation constants (*K*_{eq}) were deduced from Lineweaver-Burk plots shown on FIGURE 8. The half-life values (*t*_{1/2}) for dissociation of diabody-antigen complexes were deduced from the ratio ln2/*k*_{off}.

### Example 6: Killing tumor cells in vitro

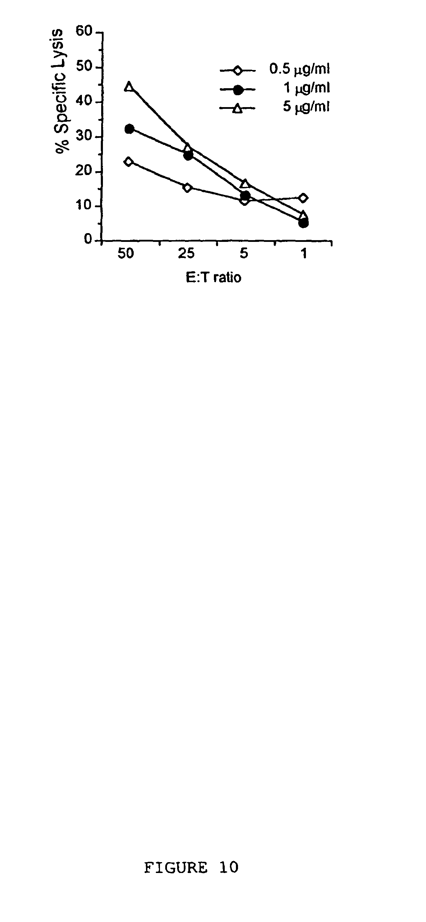

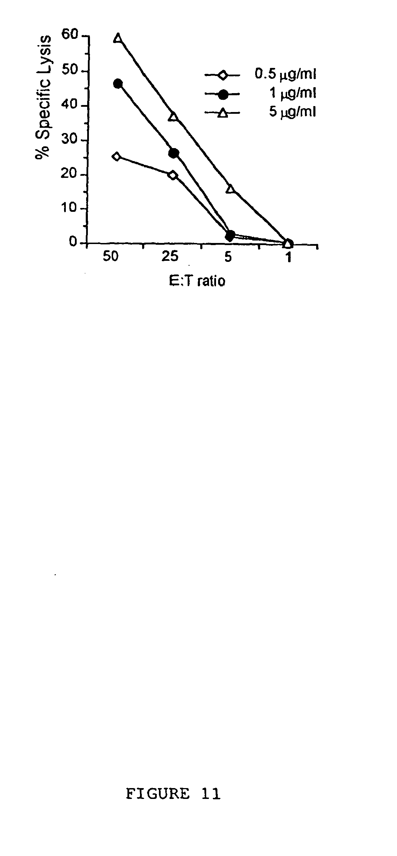

The efficacy of the CD19 x CD16 BsDb in mediating tumor cell lysis by human peripheral blood lymphocytes (PBL) or NK cells was determined using the JAM test, which is based on measuring DNA fragmentation in the target cell as a result of apoptosis (Matzinger et al., 1991, *J. Immunol. Meth.* 145, 185-192). The CD19-expressing Burkitt's lymphoma cell line Raji was used as target cells. As effector cells, either activated human PBLs prepared as described (Kirpiyanov et al., 1998, *Int. J. Cancer* 77, 763-772), or the human NK cells were used. NK cells were isolated from peripheral blood by magnetic cell sorting using the NK cell isolation kit (Miltenyi Biotec, Bergisch Gladbach, Germany). For the cell kill assay, 10⁵ effector cells were mixed in round-bottomed microtiter plates with 10⁴ target cells labeled with [³H]-thymidine in 100 µl medium plus 50 µl of diabody sample. After incubating the plate at 37°C, 5% CO₂ for 4 h, the cells were harvested and radioactivity was measured with a liquid scintillation beta counter (LKB, Wallach, Germany). Cytotoxicity related to the apoptosis-induced DNA fragmentation was calculated as % specific killing = (*S-E*)/*S* x 100, where *E* is experimentally retained labeled DNA in the presence of killers (in cpm) and *S* is retained DNA in the absence of killers (spontaneous). Difference of means was evaluated by a paired t-test using GraphPad Prism (GraphPad Software). The death of CD19⁺ Raji cells in the presence of freshly prepared PBL from a healthy donor was specifically triggered by CD19 x CD16 BsDb in a dose-dependent manner resulting in 45% of specific killing at a BsDb concentration of 5 µg/ml and E:T ratio of 50:1 (FIGURE 10). Substitution of PBL by NK cells isolated from the blood of the same donor further increased the cytotoxic effect of CD19 x CD16 BsDb up to 60% under the same conditions (FIGURE 11).

### Example 7: Treatment of Burkittis Lymphoma in severe combined immunodeficiency (SCID) mice

The SCID mice were obtained from Charles River (Sulzfeld, Germany) and kept under specific pathogen-free conditions at the Central Animal Facilities of the German Cancer Research Center. In each experiment, cohorts of five animals were used to permit accurate comparisons among differently treated groups. Mice were irradiated (300 rad) and received i.p. injections of 10 µl of anti-asialo-GM1 monoclonal antibody (Wako, Neuss, Germany) according to the manufactureris suggestions. One day later, 10⁷ Raji cells were injected s.c. dorsolaterally leading to the locally growing tumors. Treatment was started after the tumors reached a size of 5 mm in diameter (day 0). At days 0, 7, and 15, the animals received i.v. injections of either PBS (control group) or 5 x 10⁶ human PBLs. Four h after each PBL inoculation, the mice were treated either with phosphate buffered saline (PBS) or with 50 µg CD19 x CD16 BsDb administered via the tail vein. Tumor size was measured using a caliper every 2^{nd} day. Animals were followed until the s.c. tumors reached a maximal tolerated size of 15 mm in diameter and were killed by cervical dislocation. The days of sacrifice were recorded and were used for survival time analysis. The surviving animals were followed up to 60 days after the first treatment. For statistical evaluation, the follow-up duration of the tumor-treatment experiment was 30 days (end of experiment). The median survival times were estimated by the method described by Kaplan and Meier (1958, *J*. *Am. Statist. Assoc*. 53, 457-481). Differences between survival curves were compared using a logrank test (Mantel and Haenszel, 1959, *J. Natl. Cancer Inst.* 22, 719-748).

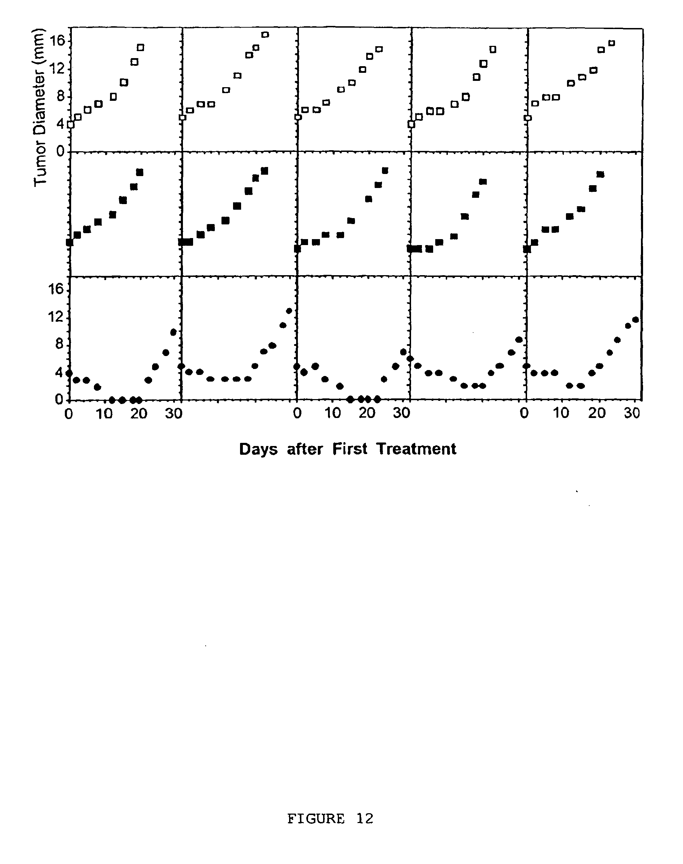

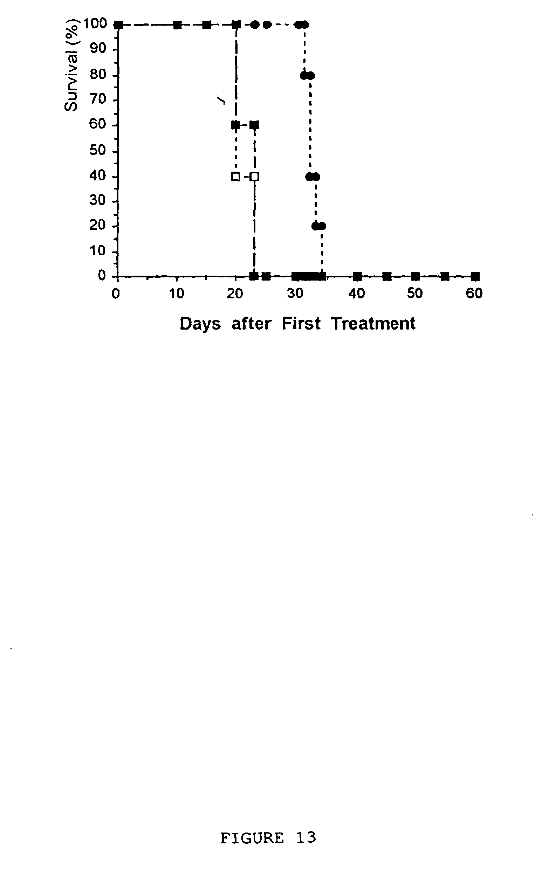

All animals in the control groups receiving PBS or PBL alone did not show any tumor suppression and developed tumors larger than 1.5 cm in diameter in less than 3 weeks (FIGURE 12). There was no significant difference between tumor growth in mice receiving PBS and mice receiving activated PBLs alone, which indicated that under the conditions used, any allogeneic reaction of the effector cells towards the tumor could be ignored. The animals were sacrificed when the tumors reached the maximum tolerated size of 15 mm in diameter. Sacrifice dates were recorded, and the median survival was calculated for each group (FIGURE 13). The median survival times were not significantly different in the control groups receiving PBS and human PBLs alone at 21.5 and 23 days, respectively (*P* = 0.4469). In contrast to control groups, all mice receiving a CD19 x CD16 BsDb demonstrated significant tumor regression. The animals receiving three injections of diabody displayed a minimal tumor size on days 15-20, when 2 out of 5 mice were tumor-free. Afterwards, however, the tumors started to grow again with comparable rates in all animals of this group (FIGURE 12). The median survival time calculated for the group receiving CD19 x CD16 BsDb (32.5 days) was significantly different from the control groups (*P* < 0.01). These *in vivo* data clearly confirms the strong antitumor effect of CD19 x CD16 BsDb, which recruits human NK cells to the tumor target.

## Claims

1. A multivalent multimeric antibody, **characterized by** the following features:
(a) it has at least two specificities;
(b) at least one antigen-binding domain is specific to human CD19; and
(c) at least one antigen-binding domain is specific to human CD16.

2. The multivalent multimeric antibody according to claim 1, which is devoid of constant regions.

3. The multivalent multimeric antibody according to claim 2, which is a single chain Fv-antibody comprising at least four immunoglobulin variable V_{H} and V_{L} domains, either separated by peptide linkers or by no linkers.

4. The multivalent multimeric antibody according to claim 2, which is a heterodimer of two hybrid single chain Fv-antibodies, each consisting of V_{H} and V_{L} domains of different specificity against CD19 or CD16, either separated by peptide linkers or by no linkers.

5. The multivalent multimeric antibody according to claim 2, which is a homodimer of single chain Fv-antibodies comprising at least four V_{H} and V_{L} domains of different specificity against CD19 or CD16, either separated by peptide linkers or by no linkers.

6. The multivalent multimeric antibody according to any one of claims 1 to 5, wherein said antigen-binding domains mimic or correspond to V_{H} and V_{L} regions from a natural antibody.

7. The multivalent multimeric antibody according to claim 6, wherein said natural antibody is a monoclonal antibody, synthetic antibody, or humanized antibody.

8. The multivalent multimeric antibody according to claim 4, wherein
(a) the first hybrid single chain Fv-antibody is V_{H}16-V_{L}19 and the second hybrid single chain Fv-antibody is V_{H}19-V_{L}16; or
(b) the first hybrid single chain Fv-antibody is V_{L}16-V_{H}19 and the second hybrid single chain Fv-antibody is V_{L}19-V_{H}16.

9. The multivalent multimeric antibody according to any one of claims 4 to 8, wherein said peptide linker comprises 0 to 12 amino acids.

10. The multivalent multimeric antibody according to claim 9, wherein said peptide linker comprises 10 amino acids.

11. The multivalent multimeric antibody to any one of claims 4 to 10, wherein the variable V_{H} or V_{L} domains are shortened by at least one amino acid residue at their N- and/or C-terminus.

12. The multivalent multimeric antibody according to claim 8, wherein two hybrid single chain Fv-antibodies are connected via a peptide linker.

13. The multivalent multimeric antibody according to claim 12, wherein said peptide linker has a length of 0-30 amino acids.

14. The multivalent multimeric antibody according to claim 13, wherein said peptide linker has a length of 12 amino acids.

15. The multivalent multimeric antibody according to any one of claims 4 to 14, wherein said peptide linkers comprise alanine, glycine, serine and proline residues.

16. The multivalent multimeric antibody according to any one of claims 4 to 15, wherein the non-covalent binding of at least one pair of V-domains is strengthened by at least one disulfide bridge.

17. The multivalent multimeric antibody according to any one of claims 4 to 16, wherein at least one monomer is linked to a an effector molecule having a conformation suitable for biological actvity or selective binding to a solid support, a biologically active substance, a chemical agent, a peptide, a protein or a drug.

18. A polynucleotide, which encodes a multivalent multimeric antibody of any one of claims 1 to 17.

19. An expression vector comprising the polynucleotide of claim 18.

20. The expression vector of claim 19, which is pSKID19x16 DSM 14529.

21. A host cell containing the expression vector of claim 19 or 20.

22. A process for the preparation of a multivalent multimeric antibody according to any one of claims 2 to 17, wherein (a) DNA sequences encoding the peptide linkers are ligated with the DNA sequences encoding the variable domains such that the peptide linkers connect the variable domains resulting in the formation of a DNA sequence encoding a monomer of the multivalent multimeric antibody and (b) the DNA sequences encoding the various monomers are expressed in a suitable expression system.

23. A composition containing the multivalent multimeric antibody of any one of claims 1 to 17, the polynucleotide of claim 18 or the expression vector of claim 19 or 20.

24. The composition of claim 23, which is a pharmaceutical composition optionally further comprising a pharmaceutically acceptable carrier or a diagnostic composition optionally further comprising suitable means for detection.

25. Use of the multivalent multimeric antibody of any of claims 1 to 17, the polynucleotide of claim 18 or the expression vector of claim 19 or 20 for the preparation of a pharmaceutical composition for treatment of B-cell malignancies, B-cell mediated autoimmune diseases or the depletion of B-cells.

26. Use according to claim 25, wherein said B-cell malignancy is non-Hodgkin lymphoma.

27. Use of the polynucleotide of claim 18 or the expression vector of claim 19 or 20 for the preparation of a composition for gene therapy.

28. Diagnostic kit, comprising:
(a) a multivalent multimeric antibody according to any one of claims 1 to 17; and/or
(b) an expression vector according to claim 19 or 20.

## Patentansprüche

1. Multivalenter multimerer Antikörper, **gekennzeichnet durch** die folgenden Merkmale:
(a) er weist mindestens zwei Spezifitäten auf;
(b) mindestens eine antigenbindende Domäne ist spezifisch für humanes CD19; und
(c) mindestens eine antigenbindende Domäne ist spezifisch für humanes CD16.

2. Multivalenter multimerer Antikörper nach Anspruch 1, der keine konstanten Regionen aufweist.

3. Multivalenter multimerer Antikörper nach Anspruch 2, der ein einzelkettiger Fv-Antikörper ist, der mindestens vier variable V_{H} und V_{L} Immunglobulin-Domänen umfasst, die entweder durch Peptidlinker oder durch keine Linker getrennt sind.

4. Multivalenter multimerer Antikörper nach Anspruch 2, der ein Heterodimer von zwei einzelkettigen Hybrid Fv-Antikörpern ist, die jeweils aus V_{H} und V_{L} Domänen von unterschiedlicher Spezifität gegen CD19 oder CD16 bestehen, die entweder durch Peptidlinker oder durch keine Linker getrennt sind.

5. Multivalenter multimerer Antikörper nach Anspruch 2, der ein Homodimer von einzelkettigen Fv-Antikörpern ist, die mindestens vier V_{H} und V_{L} Domänen unterschiedlicher Spezifität gegen CD19 oder CD16 umfassen, die entweder durch Peptidlinker oder durch keine Linker getrennt sind.

6. Multivalenter multimerer Antikörper nach einem der Ansprüche 1 bis 5, wobei die antigenbindenden Domänen V_{H} und V_{L} Regionen eines natürlichen Antikörpers imitieren oder diesen entsprechen.

7. Multivalenter multimerer Antikörper nach Anspruch 6, wobei der natürliche Antikörper ein monoklonaler Antikörper, synthetischer Antikörper oder humanisierter Antikörper ist.

8. Multivalenter multimerer Antikörper nach Anspruch 4, wobei
(a) der erste einzelkettige Hybrid Fv-Antikörper V_{H}16-V_{L}19 ist und der zweite einzelkettige Hybrid Fv-Antikörper V_{H}19-V_{L}16 ist; oder
(b) der erste einzelkettige Hybrid Fv-Antikörper V_{L}16-V_{H}19 ist und der zweite einzelkettige Hybrid Fv-Antikörper V_{L}19-V_{H}16 ist.

9. Multivalenter multimerer Antikörper nach einem der Ansprüche 4 bis 8, wobei der Peptidlinker 0 bis 12 Aminosäuren umfasst.

10. Multivalenter multimerer Antikörper nach Anspruch 9, wobei der Peptidlinker 10 Aminosäuren umfasst.

11. Multivalenter multimerer Antikörper nach einem der Ansprüche 4 bis 10, wobei die variablen V_{H} oder V_{L} Domänen um mindestens einen Aminosäurerest an ihrem N- und/oder C-Terminus verkürzt sind.

12. Multivalenter multimerer Antikörper nach Anspruch 8, wobei zwei einzelkettige Hybrid Fv-Antikörper über einen Peptidlinker verbunden sind.

13. Multivalenter multimerer Antikörper nach Anspruch 12, wobei der Peptidlinker eine Länge von 0 - 30 Aminosäuren aufweist.

14. Multivalenter multimerer Antikörper nach Anspruch 13, wobei der Peptidlinker eine Länge von 12 Aminosäuren aufweist.

15. Multivalenter multimerer Antikörper nach einem der Ansprüche 4 bis 14, wobei die Peptidlinker Alanin-, Glycin-, Serin- und Prolinreste umfassen.

16. Multivalenter multimerer Antikörper nach einem der Ansprüche 4 bis 15, wobei die nicht kovalente Bindung von mindestens einem Paar von V-Domänen durch mindestens eine Disulfidbrücke verstärkt ist.

17. Multivalenter multimerer Antikörper nach einem der Ansprüche 4 bis 16, wobei mindestens ein Monomer mit einem Effektormolekül mit einer Konformation, die für die biologische Aktivität oder selektive Bindung an einen festen Träger, eine biologisch aktive Substanz, ein chemisches Mittel, ein Peptid, ein Protein oder ein Medikament geeignet ist, verbunden ist.

18. Polynukleotid, das einen multivalenten multimeren Antikörper nach einem der Ansprüche 1 bis 17 kodiert.

19. Expressionsvektor, umfassend das Polynukleotid nach Anspruch 18.

20. Expressionsvektor nach Anspruch 19, der pSKID19x16 DSM 14529 ist.

21. Wirtszelle, enthaltend den Expressionsvektor nach Anspruch 19 oder 20.

22. Verfahren zur Herstellung eines multivalenten multimeren Antikörpers nach einem der Ansprüche 2 bis 17, wobei (a) DNA Sequenzen, die die Peptidlinker kodieren, mit den die variablen Domänen kodierenden DNA Sequenzen ligiert sind, so dass die Peptidlinker die variablen Domänen verbinden, was zur Bildung einer DNA Sequenz führt, die ein Monomer des multivalenten multimeren Antikörpers kodiert, und (b) die DNA Sequenzen, die die verschiedenen Monomere kodieren, in einem geeigneten Expressionssystem exprimiert sind.

23. Zusammensetzung, enthaltend den multivalenten multimeren Antikörper nach einem der Ansprüche 1 bis 17, das Polynukleotid nach Anspruch 18 oder den Expressionsvektor nach Anspruch 19 oder 20.

24. Zusammensetzung nach Anspruch 23, die eine pharmazeutische Zusammensetzung, die wahlweise weiter einen pharmazeutisch annehmbaren Träger umfasst, oder eine diagnostische Zusammensetzung, die wahlweise weiter geeignete Detektionsmittel umfasst, ist.

25. Verwendung des multivalenten multimeren Antikörpers nach einem der Ansprüche 1 bis 17, des Polynukleotids nach Anspruch 18 oder des Expressionsvektors nach Anspruch 19 oder 20 zur Herstellung einer pharmazeutischen Zusammensetzung zur Behandlung von B-Zellen Malignitäten, B-Zell-vermittelten Autoimmunerkrankungen oder B-Zellenschwund.

26. Verwendung nach Anspruch 25, wobei es sich bei der B-Zellen Malignität um ein Non-Hodgkin-Lymphom handelt.

27. Verwendung des Polynukleotids nach Anspruch 18 oder des Expressionsvektors nach Anspruch 19 oder 20 zur Herstellung einer Zusammensetzung für die Gentherapie.

28. Diagnostischer Kit, umfassend:
(a) einen multivalenten multimeren Antikörper nach einem der Ansprüche 1 bis 17; und/oder
(b) einen Expressionsvektor nach Anspruch 19 oder 20.

## Revendications

1. Anticorps polyvalent, multimérique, **caractérisé par** les caractéristiques suivantes :
(a) il a au moins deux spécificités,
(b) au moins un domaine de liaison des antigènes est spécifique au CD19 humain, et
(c) au moins un domaine de liaison des antigènes est spécifique au CD16 humain.

2. Anticorps polyvalent multimérique selon la revendication 1, dépourvu de régions constantes.

3. Anticorps polyvalent multimérique selon la revendication 2, qui est un anticorps Fv simple chaîne comprenant au moins quatre domaines V_{H} et V_{L} à immunoglobuline variable, séparé soit par des peptides liants, soit par aucun liant.

4. Anticorps polyvalent multimérique selon la revendication 2, qui est un hétérodimère de deux anticorps Fv simple chaîne hybrides, chacun consistant en des domaines V_{H} et V_{L} de spécificités différentes contre le CD19 ou le CD16, séparé soit par des peptides liants soit par aucun liant.

5. Anticorps polyvalent multimérique selon la revendication 2, qui est un homodimère d'anticorps Fv simple chaîne comprenant au moins quatre domaines V_{H} et V_{L} de spécificités différentes contre le CD19 ou le CD16, séparé soit par des peptides liants soit par aucun liant.

6. Anticorps polyvalent multimérique selon l'une quelconque des revendications 1 à 5, **caractérisé en ce que** les dits domaines de liaison des antigènes imitent ou correspondent aux régions V_{H} et V_{L} provenant d'un anticorps naturel.

7. Anticorps polyvalent multimérique selon la revendication 6, **caractérisé en ce que** ledit anticorps naturel est un anticorps monoclonal, un anticorps synthétique ou un anticorps humanisé.

8. Anticorps polyvalent multimérique selon la revendication 4, **caractérisé en ce que** :
(a) le premier anticorps Fv simple chaîne hybride est V_{H}16-V_{L}19 et le second anticorps Fv simple chaîne hybride est V_{L}19-V_{H}16, ou
(b) le premier anticorps Fv simple chaîne hybride est V_{L}16-V_{H}19 et le second anticorps Fv simple chaîne hybride est V_{L}19-V_{H}16.

9. Anticorps polyvalent multimérique selon l'une quelconque des revendications 4 à 8, **caractérisé en ce que** ledit peptide liant comprend entre 0 et 12 acides aminés.

10. Anticorps polyvalent multimérique selon la revendication 9, **caractérisé en ce que** le peptide liant comprend 10 acides aminés.

11. Anticorps polyvalent multimérique selon l'une quelconque des revendications 4 à 10, **caractérisé en ce que** les domaines variables V_{H} et V_{L} sont raccourcis d'au moins un résidu d'acide aminé à leur extrémité C et/ou N terminale.

12. Anticorps polyvalent multimérique selon la revendication 8, **caractérisé en ce que** deux anticorps Fv simple chaîne hybrides sont connectés par un peptide liant.

13. Anticorps polyvalent multimérique selon la revendication 12, **caractérisé en ce que** ledit peptide liant a une longueur comprise entre 0 et 30 acides aminés.

14. Anticorps polyvalent multimérique selon la revendication 13, **caractérisé en ce que** ledit peptide liant a une longueur de 12 acides aminés.

15. Anticorps polyvalent multimérique selon l'une quelconque des revendications 4 à 14, **caractérisé en ce que** lesdits peptides liants comprennent des résidus d'alanine, de glycine, de sérine et de proline.

16. Anticorps polyvalent multimérique selon l'une quelconque des revendications 4 à 15, **caractérisé en ce que** la liaison non covalente d'au moins une paire de domaines V est renforcée par au moins un pont disulfure.

17. Anticorps polyvalent multimérique selon l'une quelconque des revendications 4 à 16, **caractérisé en ce qu'**au moins un monomère est lié à une molécule effectrice ayant une conformation appropriée à l'activité biologique ou à la liaison sélective à un support solide, à une substance biologiquement active, à un agent chimique, à un peptide, à une protéine ou à un médicament.

18. Polynucléotide, qui code pour un anticorps polyvalent multimérique de l'une quelconque des revendications 1 à 17.

19. Vecteur d'expression comprenant le polynucléotide de la revendication 18.

20. Vecteur d'expression de la revendication 19, qui est pSKID19x16(DSM 14529).

21. Cellule hôte contenant le vecteur d'expression de la revendication 19 ou 20.

22. Procédé de préparation d'un anticorps polyvalent multimérique selon l'une quelconque des revendications 2 à 17, **caractérisé en ce que** (a) les séquences d'ADN codant pour les peptides liants sont liées aux séquences d'ADN codant pour les domaines variables de façon à ce que les peptides liants connectent les domaines variables ce qui résulte en la formation d'une séquence d'ADN codant pour un monomère de l'anticorps polyvalent multimérique, et (b) les séquences d'ADN codant pour les différents monomères sont exprimées dans un système d'expression approprié.

23. Composition contenant l'anticorps polyvalent multimérique de l'une quelconque des revendications 1 à 17, le polynucléotide de la revendication 18 ou le vecteur d'expression de la revendication 19 ou 20.

24. Composition de la revendication 23, qui est une composition pharmaceutique comprenant de plus, de manière optionnelle, un porteur pharmaceutiquement acceptable ou une composition de diagnostic comprenant de plus, de manière optionnelle, des moyens de détection appropriés.

25. Utilisation de l'anticorps polyvalent multimérique de l'une quelconque des revendications 1 à 17, le polynucléotide de la revendication 18 ou le vecteur d'expression de la revendication 19 ou 20 pour la préparation d'une composition pharmaceutique destinée au traitement des malignités de cellules B, des maladies autoimmunes répandues par les cellules B ou de l'épuisement des cellules B.

26. Utilisation selon la revendication 25, **caractérisée en ce que** ladite maladie de cellules B est un lymphome qui n'est pas la maladie de Hodgkin.

27. Utilisation du polynucléotide de la revendication 18 ou du vecteur d'expression de la revendication 19 ou 20 pour la préparation d'une composition destinée à la thérapie génique.

28. Kit de diagnostic, comprenant :
(a) un anticorps multimérique polyvalent selon l'une quelconque des revendications 1 à 17, et/ou
(b) un vecteur d'expression selon la revendication 19 ou 20.


### SEQUENCE LISTING

<110> Affimed Therapeutics AG
<120> Bispecific anti-CD19 x anti-CD16 antibodies and uses thereof
<130> A 3017EU
<140> EP 01127061.8
   <141> 2001-11-14
<160> 15
<170> PatentIn version 3.1
<210> 1
   <211> 1897
   <212> DNA
   <213> Mus musculus
<400> 1
   ccccaggctt tacactttat gcttccggct cgtatgttgt gtggaattgt gagcggataa     60
   caatttcaca caggaaacag ctatgaccat gattacgaat ttctgaagaa ggagatatac    120
   atatgaaata cctattgcct acggcagccg ctggcttgct gctgctggca gctcagccgg    180
   ccatggcgca ggtgcagctg cagcagtctg gagctgagct ggtaaggcct gggacttcag    240
   tgaagatatc ctgcaaggct tctggctaca ccttcactaa ctactggcta ggttgggtaa    300
   aacagaggcc tggacatgga ctcgagtgga ttggagatat ctaccctgga ggtggttata    360
   ctaactacaa tgagaaattc aagggcaagg ccacagtgac tgcagacaca tcctccagaa    420
   ctgcctacgt gcaggtcagg agcctgacat ctgaggactc tgctgtctat ttctgtgcaa    480
   gatcggctag ctggtacttc gatgtctggg gcgcacggac tacggtcacc gtctcctcag    540
   ccaaaacaac acccaagctt ggcggtgata tcttgctcac ccaaactcca gcttctttgg    600
   ctgtgtctct agggcagagg gccaccatct cctgcaaggc cagccaaagt gttgattatg    660
   atggtgatag ttatttgaac tggtaccaac agattccagg acagccaccc aaactcctca    720
   tctatgatgc atccaatcta gtttctggga tcccacccag gtttagtggc agtgggtctg    780
   ggacagactt caccctcaac atccatcctg tggagaaggt ggatgctgca acctatcact    840
   gtcagcaaag tactgaggat ccgtggacgt tcggtggagg caccaagctg gaaatcaaac    900
   gggctgatgc tgcggccgct ggatccgaac aaaagctgat ctcagaagaa gacctaaact    960
   cacatcacca tcaccatcac taaagatctg agaaggagat atacatatga aatacctatt   1020
   gcctacggca gccgctggct tgctgctgct ggcagctcag ccggccatgg cgcaggtgca   1080
   gctgcagcag tctggggctg agctggtgag gcctgggtcc tcagtgaaga tttcctgcaa   1140
   ggcttctggc tatgcattca gtagctactg gatgaactgg gtgaagcaga ggcctggaca   1200
   gggtcttgag tggattggac agatttggcc tggagatggt gatactaact acaatggaaa 1260
   gttcaagggt aaagccactc tgactgcaga cgaatcctcc agcacagcct acatgcaact 1320
   cagcagccta gcatctgagg actctgcggt ctatttctgt gcaagacggg agactacgac 1380
   ggtaggccgt tattactatg ctatggacta ctggggtcaa ggaacctcag tcaccgtctc 1440
   ctcagccaaa acaacaccca agcttggcgg tgatatccag gctgttgtga ctcaggaatc 1500
   tgcactcacc acatcacctg gtgaaacagt cacactcact tgtcgctcaa atactgggac 1560
   tgttacaact agtaactatg ccaactgggt ccaagaaaaa ccagatcatt tattcactgg 1620
   tctaataggt cataccaaca accgagctcc aggtgttcct gccagattct caggctccct 1680
   gattggagac aaggctgccc tcaccatcac aggggcacag actgaggatg aggcaatata 1740
   tttctgtgct ctatggtata acaaccattg ggtgttcggt ggaggaacca aactgactgt 1800
   cctaggccag cccaagtctg cggccgctgg atccgaacaa aagctgatct cagaagaaga 1860
   cctaaactca catcaccatc accatcacta atctaga
<210> 2
   <211> 264
   <212> PRT
   <213> Mus musculus
<400> 2
   Gln Val Gln Leu Gln Gln Ser Gly Ala Glu Leu Val Arg Pro Gly Thr
   1 5 10 15
   Ser Val Lys Ile Ser Cys Lys Ala Ser Gly Tyr Thr Phe Thr Asn Tyr
   20 25 30
   Trp Leu Gly Trp Val Lys Gln Arg Pro Gly His Gly Leu Glu Trp Ile
   35 40 45
   Gly Asp Ile Tyr Pro Gly Gly Gly Tyr Thr Asn Tyr Asn Glu Lys Phe
   50 55 60
   Lys Gly Lys Ala Thr Val Thr Ala Asp Thr Ser Ser Arg Thr Ala Tyr
   65 70 75 80
   Val Gln Val Arg Ser Leu Thr Ser Glu Asp Ser Ala Val Tyr Phe Cys
   85 90 95
   Ala Arg Ser Ala Ser Trp Tyr Phe Asp Val Trp Gly Ala Arg Thr Thr
   100 105 110
   Val Thr Val Ser Ser Ala Lys Thr Thr Pro Lys Leu Gly Gly Asp Ile
   115 120 125
   Leu Leu Thr Gln Thr Pro Ala Ser Leu Ala Val Ser Leu Gly Gln Arg
   130 135 140
   Ala Thr Ile Ser Cys Lys Ala Ser Gln Ser Val Asp Tyr Asp Gly Asp
   145 150 155 160
   Ser Tyr Leu Asn Trp Tyr Gln Gln Ile Pro Gly Gln Pro Pro Lys Leu
   165 170 175
   Leu Ile Tyr Asp Ala Ser Asn Leu Val Ser Gly Ile Pro Pro Arg Phe
   180 185 190
   Ser Gly Ser Gly Ser Gly Thr Asp Phe Thr Leu Asn Ile His Pro Val
   195 200 205
   Glu Lys Val Asp Ala Ala Thr Tyr His Cys Gln Gln Ser Thr Glu Asp
   210 215 220
   Pro Trp Thr Phe Gly Gly Gly Thr Lys Leu Glu Ile Lys Arg Ala Asp
   225 230 235 240
   Ala Ala Ala Ala Gly Ser Glu Gln Lys Leu Ile Ser Glu Glu Asp Leu
   245 250 255
   Asn Ser His His His His His His
   260
<210> 3
   <211> 272
   <212> PRT
   <213> Mus musculus
<400> 3
   Gln Val Gln Leu Gln Gln Ser Gly Ala Glu Leu Val Arg Pro Gly Ser
   1               5                   10                  15
   Ser Val Lys Ile Ser Cys Lys Ala Ser Gly Tyr Ala Phe Ser Ser Tyr
               20                  25                  30
   Trp Met Asn Trp Val Lys Gln Arg Pro Gly Gln Gly Leu Glu Trp Ile
           35                  40                  45
   Gly Gln Ile Trp Pro Gly Asp Gly Asp Thr Asn Tyr Asn Gly Lys Phe
       50                  55                  60
   Lys Gly Lys Ala Thr Leu Thr Ala Asp Glu Ser Ser Ser Thr Ala Tyr
   65 70 75 80
   Met Gln Leu Ser Ser Leu Ala Ser Glu Asp Ser Ala Val Tyr Phe Cys
   85 90 95
   Ala Arg Arg Glu Thr Thr Thr Val Gly Arg Tyr Tyr Tyr Ala Met Asp
   100 105 110
   Tyr Trp Gly Gln Gly Thr Ser Val Thr Val Ser Ser Ala Lys Thr Thr
   115 120 125
   Pro Lys Leu Gly Gly Asp Ile Gln Ala Val Val Thr Gln Glu Ser Ala
   130 135 140
   Leu Thr Thr Ser Pro Gly Glu Thr Val Thr Leu Thr Cys Arg Ser Asn
   145 150 155 160
   Thr Gly Thr Val Thr Thr Ser Asn Tyr Ala Asn Trp Val Gln Glu Lys
   165 170 175
   Pro Asp His Leu Phe Thr Gly Leu Ile Gly His Thr Asn Asn Arg Ala
   180 185 190
   Pro Gly Val Pro Ala Arg Phe Ser Gly Ser Leu Ile Gly Asp Lys Ala
   195 200 205
   Ala Leu Thr Ile Thr Gly Ala Gln Thr Glu Asp Glu Ala Ile Tyr Phe
   210 215 220
   Cys Ala Leu Trp Tyr Asn Asn His Trp Val Phe Gly Gly Gly Thr Lys
   225 230 235 240
   Leu Thr Val Leu Gly Gln Pro Lys Ser Ala Ala Ala Gly Ser Glu Gln
   245 250 255
   Lys Leu Ile Ser Glu Glu Asp Leu Asn Ser His His His His His His
   260 265 270
<210> 4
   <211> 5
   <212> PRT
   <213> Artificial Sequence
<220>
   <223> Description of the Artificial Sequence: LacZ
<400> 4
   Met Ile Thr Asn Phe
   5
<210> 5
   <211> 22
   <212> PRT
   <213> Artificial Sequence
<220>
   <223> Description of the Artificial Sequence: PelB Leader
<400> 5
   Met Lys Tyr Leu Leu Pro Thr Ala Ala Ala Gly Leu Leu Leu Leu Ala
   5 10 15
   Ala Gln Pro Ala Met Ala
   20
<210> 6
   <211> 10
   <212> PRT
   <213> Artificial Sequence
<220>
   <223> Description of the Artificial Sequence: Peptide Linker
<400> 6
   Ser Ala Lys Thr Thr Pro Lys Leu Gly Gly
   5 10
<210> 7
   <211> 12
   <212> PRT
   <213> Artificial Sequence
<220>
   <223> Description of the Artificial Sequence: Peptide Linker
<400> 7
   Arg Ala Asp Ala Ala Ala Ala Gly Gly Pro Gly Ser
   5 10
<210> 8
   <211> 32
   <212> DNA
   <213> Artificial Sequence
<220>
   <223> Description of the Artificial Sequence: Primer
<400> 8
   gatatacata tgaaatacct attgcctacg gc 3 2
<210> 9
   <211> 47
   <212> DNA
   <213> Artificial Sequence
<220>
   <223> Description of the Artificial Sequence: Primer
<400> 9
   cgaattctta agttagcaca ggcctctaga gacacacaga tctttag 47
<210> 10
   <211> 28
   <212> DNA
   <213> Artificial Sequence
<220>
   <223> Description of the Artificial Sequence: Primer
<400> 10
   caccctggcg cccaatacgc aaaccgcc 28
<210> 11
   <211> 46
   <212> DNA
   <213> Artificial Sequence
<220>
   <223> Description of the Artificial Sequence: Primer
<400> 11
   ggtatttcat atgtatatct ccttcttcag aaattcgtaa tcatgg 46
<210> 12
   <211> 52
   <212> DNA
   <213> Artificial Sequence
<220>
   <223> Description of the Artificial Sequence: Primer
<400> 12
   cgaattctta agaaggagat atacatatga aaaagtggtt attagctgca gg 52
<210> 13
   <211> 40
   <212> DNA
   <213> Artificial Sequence
<220>
   <223> Description of the Artificial Sequence: Primer
<400> 13
   cgaattctcg agcattattt aacctgtttc agtacgtcgg 40
<210> 14
   <211> 52
   <212> DNA
   <213> Artificial Sequence
<220>
   <223> Description of the Artificial Sequence: Primer
<400> 14
   gcacacagat ctgagaagga gatatacata tgaaatacct attgcctacg gc 52
<210> 15
   <211> 34
   <212> DNA
   <213> Artificial Sequence
<220>
   <223> Description of the Artificial Sequence: Primer
<400> 15
   ggtcgacgtt aaccgacaaa caacagataa aacg 34